# EUROPEAN PATENT APPLICATION

(11) **EP 1 669 358 A1**
(43) Date of publication of application: **14.06.2006**
(21) Application number: 04292898.6
(22) Date of filing: 07.12.2004
(51) Int. Cl.: C07D 493/06, C07K 16/30, A61K 39/395, A61P 35/00

(54) **Cytotoxic agents comprising new taxanes**

(71) Applicant: Aventis Pharma S.A., 92160 Antony (FR)
(72) Inventor: Miller, Michael L., Framingham, MA 01701 (US); Chari, Ravi V.J., Newton, MA 02461 (US); Baloglu, Erkan, Stoneham, MA 02180 (US); Commerçon, Alain, 94400 Vitry sur Seine (FR)
(74) Representative: Le Pennec, Magali

(57) **Abstract**

The present invention relates to novel cytotoxic agents and their therapeutic use. More specifically, the invention relates to novel cytotoxic agents comprising taxanes and their therapeutic use. These novel cytotoxic agents have therapeutic use as a result of delivering the taxanes to a specific cell population in a targeted fashion by chemically linking the taxane to a cell binding agent.

## Description

### FIELD OF THE INVENTION

The present invention relates to novel cytotoxic agents and their therapeutic use. More specifically, the invention relates to novel cytotoxic agents comprising taxanes and their therapeutic use. These novel cytotoxic agents have therapeutic use as a result of delivering the taxanes to a specific cell population in a targeted fashion by chemically linking the taxane to a cell binding agent.

### BACKGROUND OF THE INVENTION

Many reports have appeared on the attempted specific targeting of tumor cells with monoclonal antibody-drug conjugates (Sela et al, in *Immunoconjugates* 189-216 (C. Vogel, ed. 1987); Ghose et al, in *Targeted Drugs* 1-22 (E. Goldberg, ed. 1983); Diener et al, in *Antibody mediated delivery systems* 1-23 (J. Rodwell, ed. 1988); Pietersz et al, in *Antibody mediated delivery systems* 25-53 (J. Rodwell, ed. 1988); Bumol et al, in *Antibody mediated delivery systems* 55-79 (J. Rodwell, ed. 1988). All references and patents cited herein are incorporated by reference.

Cytotoxic drugs such as methotrexate, daunorubicin, doxorubicin, vincristine, vinblastine, melphalan, mitomycin C, and chlorambucil have been conjugated to a variety of murine monoclonal antibodies. In some cases, the drug molecules were linked to the antibody molecules through an intermediary carrier molecule such as serum albumin (Garnett et al, 46 *Cancer Res.* 2407-2412 (1986); Ohkawa et al 23 *Cancer Immunol. Immunother.* 81-86 (1986); Endo et al, 47 *Cancer Res.* 1076-1080 (1980)), dextran (Hurwitz et al, 2 *Appl. Biochem.* 25-35 (1980); Manabi et al, 34 *Biochem. Pharmacol.* 289-291 (1985); Dillman et al, 46 *Cancer Res.* 4886-4891 (1986); Shoval et al, 85 *Proc. Natl. Acad. Sci.* 8276-8280 (1988)), or polyglutamic acid (Tsukada et al, 73 J. *Natl. Canc. Inst.* 721-729 (1984); Kato et al 27 J. *Med.* Chem. 1602-1607 (1984); Tsukada et al, 52 *Br. J. Cancer* 111-116 (1985)).

A wide array of linker technologies has been employed for the preparation of such immunoconjugates and both cleavable and non-cleavable linkers have been investigated. In most cases, the full cytotoxic potential of the drugs could only be observed, however, if the drug molecules could be released from the conjugates in unmodified form at the target site.

One of the cleavable linkers that has been employed for the preparation of antibody-drug conjugates is an acid-labile linker based on cis-aconitic acid that takes advantage of the acidic environment of different intracellular compartments such as the endosomes encountered during receptor mediated endocytosis and the lysosomes. Shen and Ryser introduced this method for the preparation of conjugates of daunorubicin with macromolecular carriers (102 *Biochem. Biophys.* Res. *Commun.* 1048-1054 (1981)). Yang and Reisfeld used the same technique to conjugate daunorubicin to an anti-melanoma antibody (80 *J. Natl. Canc. Inst.* 1154-1159 (1988)). Dillman et al. also used an acid-labile linker in a similar fashion to prepare conjugates of daunorubicin with an anti-T cell antibody (48 *Cancer* Res. 6097-6102 (1988)).

An alternative approach, explored by Trouet et al, involved linking daunorubicin to an antibody via a peptide spacer arm (79 Proc. *Natl.* Acad. *Sci.* 626-629 (1982)). This was done under the premise that free drug could be released from such a conjugate by the action of lysosomal peptidases.

*In vitro* cytotoxicity tests, however, have revealed that antibody-drug conjugates rarely achieved the same cytotoxic potency as the free unconjugated drugs. This suggested that mechanisms by which drug molecules are released from the antibodies are very inefficient. In the area of immunotoxins, conjugates formed via disulfide bridges between monoclonal antibodies and catalytically active protein toxins were shown to be more cytotoxic than conjugates containing other linkers. See, Lambert et al, 260 *J*. *Biol.* Chem. 12035-12041 (1985); Lambert et al, in *Immunotoxins* 175-209 (A. Frankel, ed. 1988); Ghetie et al, 48 *Cancer Res.* 2610-2617 (1988). This was attributed to the high intracellular concentration of glutathione contributing to the efficient cleavage of the disulfide bond between an antibody molecule and a toxin. Despite this, there are only a few reported examples of the use of disulfide bridges for the preparation of conjugates between drugs and macromolecules. Shen et al (260 J. *Biol. Chem.* 10905-10908 (1985)) described the conversion of methotrexate into a mercaptoethylamide derivative followed by conjugation with poly-D-lysine via a disulfide bond. Another report described the preparation of a conjugate of the trisulfide containing toxic drug calicheamycin with an antibody (Hinman et al., 53 *Cancer Res.* 3336-3342 (1993)).

One reason for the lack of disulfide linked antibody-drug conjugates is the unavailability of cytotoxic drugs possessing a sulfur atom containing moiety that can be readily used to link the drug to an antibody via a disulfide bridge. Furthermore, chemical modification of existing drugs is difficult without diminishing their cytotoxic potential.

Another major drawback with existing antibody-drug conjugates is their inability to deliver a sufficient concentration of drug to the target site because of the limited number of targeted antigens and the relatively moderate cytotoxicity of cancerostatic drugs like methotrexate, daunorubicin, and vincristine. In order to achieve significant cytotoxicity, linkage of a large number of drug molecules, either directly to the antibody or through a polymeric carrier molecule, becomes necessary. However, such heavily modified antibodies often display impaired binding to the target antigen and fast *in vivo* clearance from the blood stream.

In spite of the above described difficulties, useful cytotoxic agents comprising cell binding moieties and the group of cytotoxic drugs known as maytansinoids have been reported (USP 5,208,020, USP 5,416,064, and R. V. J. Chari, 31 *Advanced Drug Delivery Reviews* 89-104 (1998)). Similarly, useful cytotoxic agents comprising cell binding moieties and analogues and derivatives of the potent antitumor antibotic CC-1065 have also been reported (USP 5,475,092 and USP 5,585,499).

Paclitaxel (Taxol), a cytotoxic natural product, and docetaxel (Taxotere), a semi-synthetic derivative, are widely used in the treatment of cancer. These compounds belong to the family of compounds called taxanes. Taxanes are mitotic spindle poisons that inhibit the depolymerization of tubulin, resulting in an increase in the rate of microtubule assembly and cell death. While docetaxel and paclitaxel are useful agents in the treatment of cancer, their antitumor activity is limited because of their non-specific toxicity towards abnormal cells.

Further, compounds like paclitaxel and docetaxel themselves are not sufficiently potent to be used in conjugates of cell binding agents. Recently, a few new docetaxel analogs with greater potency than either docetaxel or paclitaxel have been described (Ojima et al., 39, *J. Med. Chem.* 3889-3896 (1996)). However, these compounds lack a suitable functionality that allows linkage via a cleavable bond to cell binding agents.

Accordingly, a method of treating diseases with taxanes wherein their side effects are reduced without compromising their cytotoxicity is greatly needed.

US 6,436,931, US 6,372,738 and US 6,340,701 described taxanes linked by a disulfide bridge to the monoclonal antibody. Those taxanes seem to be not sufficiently potent to be used.

### SUMMARY OF THE INVENTION

As disclosed in a first embodiment, one object of the present invention is to provide taxanes that are highly toxic and that can still be effectively used in the treatment of many diseases.

Another object of the present invention is to provide novel taxanes.

in a second embodiment the invention provides a cytotoxic agent comprising one or more taxanes linked to a cell binding agent.

In a third embodiment, the present invention provides a therapeutic composition comprising :
(A) an effective amount of one or more taxanes linked to a cell binding agent, and
(B) a pharmaceutically acceptable carrier, diluent, or excipient

In a fourth embodiment, the present invention provides a method of killing selected cell populations comprising contacting target cells or tissue containing target cells, with a cytotoxic amount of a cytotoxic agent comprising one or more taxanes linked to a cell binding agent.

### DETAILED DESCRIPTION OF THE INVENTION

This invention is based on the synthesis of novel taxanes that retain high cytotoxicity and that can be effectively linked to cell binding agents. It has previously been shown that the linkage of highly cytotoxic drugs to antibodies using a cleavable link, such as a disulfide bond, ensures the release of fully active drugs inside the cell, and such conjugates are cytotoxic in an antigen specific manner (US 6,340,701 ; US 6,372,738 ; US 6,436,931). However, the art reveals that it is extremely difficult to modify existing drugs without diminishing their cytotoxic potential. The disclosed invention overcomes this problem by modifying the disclosed taxanes with chemical moieties. As a result, the disclosed novel taxanes preserve, and in some cases could even enhance, the cytotoxic potency of known taxanes. The cell binding agent-taxane complexes permit the full measure of the cytotoxic action of the taxanes to be applied in a targeted fashion against unwanted cells only, therefore, avoiding side effects due to damage to non-targeted healthy cells. Thus, the invention provides useful agents for the elimination of diseased or abnormal cells that are to be killed or lysed such as tumor cells (particularly solid tumor cells).

The cytotoxic agent according to the present invention comprises one or more taxanes linked to a cell binding agent via a linking group. The linking group is part of a chemical moiety that is covalently bound to a taxane through conventional methods. In a preferred embodiment, the chemical moiety can be covalently bound to the taxane via an ester linkage.

The taxanes useful in the present invention have the formula (I) shown below : 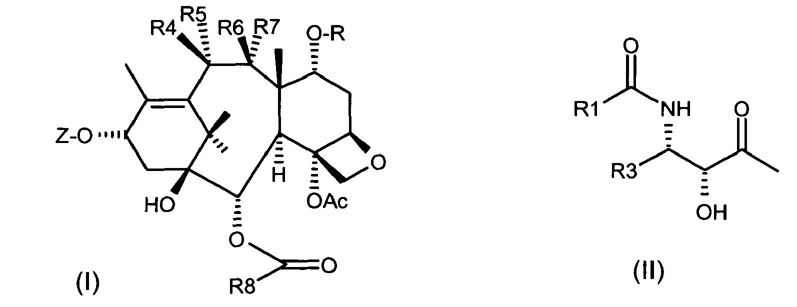
**Z** = H or a radical of formula **II**
**R**_{**1**} is a linker or an optionally substituted aryl or heterocyclic radical, alkyl
having from 1 to 10 carbon atoms, alkenyl or alkynyl having from 2 to 10 carbon atoms, cycloalkyl or cycloalkenyl having from 3 to 10 carbon atoms, -OR₂ or a carbamate formed from any of said alkyl having from 1 to 10 carbon atoms, alkenyl or alkynyl having from 2 to 10 carbon atoms, cycloalkyl or cycloalkenyl having from 3 to 10 carbon atoms, or optionally substituted aryl or heterocyclyl radical. Preferably R₁ is -OR₂ or an optionally substituted aryl or heterocyclic radical
**R**_{**2**} is an alkyl radical having from 1 to 10 carbon atoms, alkenyl or alkynyl having from 2 to 10 carbon atoms, cycloalkyl or cycloalkenyl having from 3 to 10 carbon atoms, optionally substituted aryl or heterocyclic radical. Preferably R₂ is an alkyl group and more preferably a substituted alkyl group such as a terbutyl group.
**R**_{**3**} is a linker or an optionally substituted aryl or heterocyclic radical, alkyl having from 1 to 10 carbon atoms, alkenyl or alkynyl having from 2 to 10 carbon atoms, cycloalkyl or cycloalkenyl having from 3 to 10 carbon atoms
**R**_{**4**} is a linker, H, a hydroxy radical, an alkoxy, an alkenyloxy, an optionally substituted alkanoyloxy, aroyloxy, alkenoyloxy, alkynoyloxy, cycloalkanoyloxy, alkoxyacetyl, alkylthioacetyl, alkyloxycarbonyloxy, cycloalkyloxy, cycloalkenyloxy, carbamoyloxy, alkylcarbamoyloxy, or dialkylcarbamoyloxy, a heterocyclic or aryl ether, ester or carbamate, or, a linear, branched, or cyclic alkyl or alkenyl ester or ether having from 1 to 10 carbon atoms or a carbamate of the formula -OCOX,
   wherein X is a nitrogen-containing heterocycle such as unsubstituted or substituted piperidino, morpholino, piperazino, N-methylpiperazino, or a carbamate of the formula -OCON**R**_{**9**}**R**_{**10**}, wherein **R**_{**9**} and **R**_{**10**} are the same or different and are H, linear, branched, or cyclic alkyl having from 1 to 10 atoms or unsubstituted or substituted aryl having from 1 to 10 carbon atoms. Preferably R₄ is a linker or an alkanoyloxy radical.
**R**_{**5**} or **R**_{**7**} is H
**R**_{**6**} is H
**R**_{**7**} or **R**_{**5**} and **R** form a bond (cyclic ether)
**R**_{**8**} = optionally substituted aryl or heterocyclic radical

The present invention will be more completely described with its 3 major embodiments.

### Embodiment 1: R₄ is the linker

**Z** = H or radical of formula **II**

**R**_{**1**} is an optionally substituted aryl or heterocyclic radical, alkyl having from 1 to 10 carbon atoms, alkenyl or alkynyl having from 2 to 10 carbon atoms, cycloalkyl or cycloalkenyl having from 3 to 10 carbon atoms, **-OR**_{**2**} or a carbamate formed from any of said alkyl having from 1 to 10 carbon atoms, alkenyl or alkynyl having from 2 to 10 carbon atoms, cycloalkyl or cycloalkenyl having from 3 to 10 carbon atoms, or optionally substituted aryl or heterocyclyl radical

**R**_{**2**} is alkyl having from 1 to 10 carbon atoms, alkenyl or alkynyl having from 2 to 10 carbon atoms, cycloalkyl or cycloalkenyl having from 3 to 10 carbon atoms, optionally substituted aryl or heterocyclic radical.

Preferably, **R**_{**1**} is t-butoxy, crotyl, dimethylacrylyl, isobutenyl, hexenyl, cyclopentenyl, cyclohexenyl, furyl, pyrollyl, thienyl, thiazolyl, imidazolyl, pyridyl, morpholino, piperidino, piperazino, oxazolyl, indolyl, benzofuranyl or benzothienyl.

More preferably, **R**_{**1**} is t-butoxy, isobutenyl, crotyl, dimethyacrylyl, thienyl, thiazolyl or furyl

**R**_{**3**} is optionally substituted aryl or heterocyclic radical, alkyl having from 1 to 10 carbon atoms, alkenyl or alkynyl having from 2 to 10 carbon atoms, cycloalkyl or cycloalkenyl having from 3 to 10 carbon atoms

Preferably, **R**_{**3**} is crotyl, dimethylacrylyl, propenyl, isobutenyl, hexenyl, cyclopentenyl, cyclohexenyl, furyl, pyrollyl, thienyl, thiazolyl, pyridyl, oxazolyl, indolyl, benzofuranyl or benzothienyl.

More preferably, **R**_{**3**} is iso-butenyl, crotyl, dimethyacrylyl, thienyl, thiazolyl, pyridyl, tert-butyl, or furyl.

**R**_{**4**} is the linking group.

Suitable linking groups are well known in the art and include disulfide groups, thioether groups, acid labile groups, photolabile groups, peptidase labile groups and esterase labile groups. Preferred are disulfide groups and thioether groups.

When the linking group is a thiol- or disulfide-containing group, the side chain carrying the thiol or disulfide group can be linear or branched, aromatic or heterocyclic. One of ordinary skill in the art can readily identify suitable side chains.

Specific examples of the thiol- or disulfide-containing substituents include -O(CR₁₃R₁₄)ₘ(CR₁₅R₁₆)ₙ(OCH₂CH₂)_{y}SZ', -OCO(CR₁₃R₁₄)ₘ(CR₁₅R₁₆)ₙ (OCH₂CH₂)_{y} SZ', -O(CR₁₃R₁₄)ₘ (CR₁₇=CR₁₈)(CR₁₅R₁₆)ₘ (OCH₂CH₂)_{y}SZ', -OCO-(CR₁₃R₁₄)ₘ (CR₁₇=CR₁₈)(CR₁₅R₁₆)ₘ (OCH₂CH₂)_{y}SZ', -OCONR₁₂(CR₁₃R₁₄)ₘ(CR₁₅R₁₆)ₙ (OCH₂CH₂)_{y}SZ', -OCO-phenyl-X'SZ', -OCO-furyl-X'SZ', -OCO-oxazolyl-X'SZ', -OCO-thiazolyl-X'SZ', -OCO-thienyl-X'SZ', -OCO-imidazolyl-X'SZ', -OCO-morpholino-X'SZ', -OCO-piperazino-X'SZ', -OCO-piperidino-X'SZ', and -OCO-N-methylpiperazino-X'SZ', or -OCO-N-methylpiperazino-X'SZ', wherein:

Z' is H or SR',
wherein X' is a direct link or a linear alkyl or branched alkyl having from 1-10 carbon atoms or a polyethylene glycol spacer with 2 to 20 repeating ethylene oxy units;

R' and R₁₂ are the same or different and are linear alkyl, branched alkyl or cyclic alkyl having from 1 to 10 carbon atoms, or simple or substituted aryl or heterocyclic, and R₁₂ can in addition be H,

R₁₃, R₁₄, R₁₅ and R₁₆ are the same or different and are H or a linear or branched alkyl having from 1 to 4 carbon atoms,

R₁₇ and R₁₈ are H or alkyll,
n is an integer of 1 to 10,
m is an integer from 1 to 10 and can also be 0,
y is an integer from 1 to 20 and can also be 0.

**R**_{**5**} or **R**_{**7**} is **HR**_{**6**} is H

**R**_{**7**} or **R**_{**5**} and **R** form a bond (cyclic ether)

**R**_{**8**} is an optionally substituted aryl or heterocyclic radical

Preferably, **R**_{**8**} is 3-methoxyphenyl, 3-chlorophenyl, 2,5-dimethoxyphenyl, furyl, pyrollyl, thienyl, thiazolyl, imidazolyl, pyridyl, indolyl, oxazolyl, benzofuranyl or benzothenyl.

### Embodiment 2: R₁ is the linker

Z is a radical of formula II

**R**_{**1**} is the linking group.

Suitable linking groups are well known in the art and include disulfide groups, thioether groups, acid labile groups, photolabile groups, peptidase labile groups and esterase labile groups. Preferred are disulfide groups and thioether groups.

When the linking group is a thiol- or disulfide-containing group, the side chain carrying the thiol or disulfide group can be linear or branched, aromatic or heterocyclic. One of ordinary skill in the art can readily identify suitable side chains.

Specific examples of the thiol- or disulfide-containing substituents include -(CR₁₃R₁₄)ₘ(CR₁₅R₁₆)ₙ(OCH₂CH₂)_{y}SZ', -O(CR₁₃R₁₄)ₘ(CR₁₅R₁₆)ₙ (OCH₂CH₂)_{y} SZ', -(CR₁₃R₁₄)ₘ (CR₁₇=CR₁₈)(CR₁₅R₁₆)ₘ (OCH₂CH₂)_{y}SZ', -O-(CR₁₃R₁₄)ₘ (CR₁₇=CR₁₈)(CR₁₅R₁₆)ₘ (OCH₂CH₂)_{y}SZ', -NR₁₂(CR₁₃R₁₄)ₘ(CR₁₅R₁₆)ₙ (OCH₂CH₂)_{y}SZ', phenyl-X'SZ', furyl-XSZ', oxazolyl-X'SZ', thiazolyl-X'SZ', thienyl-X'SZ', imidazolyl-X'SZ', morpholino-X'SZ', -piperazino-X'SZ', piperidino-XSZ', -furyl-X'SZ', - thienyl-X'SZ', -thiazolyl-X'SZ' and --N-methylpiperazino-X'SZ', -morpholino-X'SZ', -piperazino-X'SZ', -piperidino-X'SZ', or -N-methylpiperazino-X'SZ', wherein:
Z' is H or SR',
wherein X' is a direct link or a linear alkyl or branched alkyl having from 1-10 carbon atoms or a polyethylene glycol spacer with 2 to 20 repeating ethylene oxy units;
R' and R₁₂ are the same or different and are linear alkyl, branched alkyl or cyclic alkyl having from 1 to 10 carbon atoms, or simple or substituted aryl or heterocyclic, and R₁₂ can in addition be H,
R₁₃, R₁₄, R₁₅ and R₁₆ are the same or different and are H or a linear or branched alkyl having from 1 to 4 carbon atoms,
R₁₇ and R₁₈ are H or alkyl,
   n is an integer of 1 to 10,
   m is an integer from 1 to 10 and can also be 0,
   y is an integer from 1 to 20 and can also be 0.
**R**_{**3**} is optionally substituted aryl or heterocyclic radical, alkyl having from 1 to 10 carbon atoms, alkenyl or alkynyl having from 2 to 10 carbon atoms, cycloalkyl or cycloalkenyl having from 3 to 10 carbon atoms

Preferably, **R**_{**3**} is crotyl, dimethylacrylyl, propenyl, isobutenyl, hexenyl, cyclopentenyl, cyclohexenyl, furyl, pyrollyl, thienyl, thiazolyl, imidazolyl, pyridyl, oxazolyl, indolyl, benzofuranyl or benzothienyl.

More preferably, **R**_{**3**} is iso-butenyl, crotyl, dimethyacrylyl, thienyl, thiazolyl, pyridyl, tert-butyl, or furyl.

**R**_{**4**} is H, a hydroxy radical, an alkoxy, an alkenyloxy, an optionally substituted alkanoyloxy, aroyloxy, alkenoyloxy, alkynoyloxy, cycloalkanoyloxy, alkoxyacetyl, alkylthioacetyl, alkyloxycarbonyloxy, cycloalkyloxy, cycloalkenyloxy, carbamoyloxy, alkylcarbamoyloxy or dialkylcarbamoyloxy, a heterocyclic or aryl ether, ester or carbamate, or, a linear, branched, or cyclic alkyl or alkenyl ester or ether having from 1 to 10 carbon atoms or a carbamate of the formula -OCOX, wherein X is a nitrogen-containing heterocycle such as unsubstituted or substituted piperidino, morpholino, piperazino, N-methylpiperazino, or a carbamate of the formula -OCON**R**_{**9**}**R**_{**10**}**,** wherein **R**_{**9**} and **R**_{**10**} are the same or different and are H, linear, branched, or cyclic alkyl having from 1 to 10 atoms or simple or substituted aryl having from 5 to 10 carbon atoms;

**R**_{**5**} or **R**_{**7**} is H

**R**_{**6**} is H

**R**_{**7**} or **R**_{**5**} and **R** form a bond (cyclic ether)

**R**_{**8**} is an optionally substituted aryl or heterocyclic radical

Preferably, **R**_{**8**} is 3-methoxyphenyl, 3-chlorophenyl, 2,5-dimethoxyphenyl, furyl, pyrollyl, thienyl, thiazolyl, oxazolyl, imidazolyl, pyridyl, indolyl, benzofuranyl or benzothienyl.

### Embodiment 3: R₃ is the linker

**Z** is a radical of formula **II**

**R**_{**1**} is an optionally substituted aryl or heterocyclic radical, alkyl having from 1 to 10 carbon atoms, alkenyl or alkynyl having from 2 to 10 carbon atoms, cycloalkyl or cycloalkenyl having from 3 to 10 carbon atoms, -OR₂ or a carbamate formed from any of said alkyl having from 1 to 10 carbon atoms, alkenyl or alkynyl having from 2 to 10 carbon atoms, cycloalkyl or cycloalkenyl having from 3 to 10 carbon atoms, or optionally substituted aryl or heterocyclyl radical

**R**_{**2**} is alkyl radical having from 1 to 10 carbon atoms, alkenyl or alkynyl having from 2 to 10 carbon atoms, cycloalkyl or cycloalkenyl having from 3 to 10 carbon atoms, optionally substituted aryl or heterocyclic radical

Preferably, **R**_{**1**} is t-butoxy, crotyl, dimethylacrylyl, isobutenyl, hexenyl, cyclopentenyl, cyclohexenyl, furyl, pyrollyl, thienyl, thiazolyl, imidazolyl, pyridyl, morpholino, piperidino, piperazino, oxazolyl, indolyl, benzofuranyl or benzothienyl.

More preferably, **R**_{**1**} is t-butoxy, isobutenyl, crotyl, dimethyacrylyl, thienyl, thiazolyl or furyl.

**R**_{**3**} is the linking group.

Suitable linking groups are well known in the art and include disulfide groups, thioether groups, acid labile groups, photolabile groups, peptidase labile groups and esterase labile groups. Preferred are disulfide groups and thioether groups.

When the linking group is a thiol- or disulfide-containing group, the side chain carrying the thiol or disulfide group can be linear or branched, aromatic or heterocyclic. One of ordinary skill in the art can readily identify suitable side chains.

Specific examples of the thiol- or disulfide-containing substituents include -(CR₁₃R₁₄)ₘ(CR₁₅R₁₆)ₙ(OCH₂CH₂)_{y}SZ', -(CR₁₃R₁₄)ₘ (CR₁₇=CR₁₈)(CR₁₅R₁₆)ₘ (OCH₂CH₂)_{y}SZ', phenyl-X'SZ', furyl-X'SZ', oxazolyl-X'SZ', thiazolyl-X'SZ', thienyl-X'SZ', imidazolyl-X'SZ', wherein:
Z' is H or SR',
Wherein X' is a direct link or a linear alkyl or branched alkyl having from 1-10 carbon atoms or a polyethylene glycol spacer with 2 to 20 repeating ethylene oxy units ;
R' is linear alkyl, branched alkyl or cyclic alkyl having from 1 to 10 carbon atoms, or simple or substituted aryl or heterocyclic,
R₁₃, R₁₄, R₁₅ and R₁₆ are the same or different and are H or a linear or branched alkyl having from 1 to 4 carbon atoms,
R₁₇ and R₁₈ are H or alkyl,
   n is an integer of 1 to 10,
   m is an integer from 1 to 10 and can also be 0,
   y is an integer from 1 to 20 and can also be 0.
**R**_{**4**} is H, a hydroxy radical, an alkoxy, an alkenyloxy, an optionally substituted alkanoyloxy, aroyloxy, alkenoyloxy, alkynoyloxy, cycloalkanoyloxy, alkoxyacetyl, alkylthioacetyl, alkyloxycarbonyloxy, cycloalkyloxy, cycloalkenyloxy, carbamoyloxy, alkylcarbamoyloxy, or dialkylcarbamoyloxy, a heterocyclic or aryl ether, ester or carbamate, or, a linear, branched, or cyclic alkyl or alkenyl ester or ether having from 1 to 10 carbon atoms or a carbamate of the formula -OCOX, wherein X is a nitrogen-containing heterocycle such as unsubstituted or substituted piperidino, morpholino, piperazino, N-methylpiperazino, or a carbamate of the formula -OCONR₉R₁₀, wherein **R**_{**9**} and **R**_{**10**} are the same or different and are H, linear, branched, or cyclic alkyl having from 1 to 10 atoms or simple or substituted aryl having from 5 to 10 carbon atoms ;
**R**_{**5**} or **R**_{**7**} is H
**R**_{**6**} is H
**R**_{**7**} or **R**_{**5**} and **R** form a bond (cyclic ether)
**R**_{**8**} is an optionally substituted aryl or heterocyclic radical

Preferably, **R**_{**8**} is 3-methoxyphenyl, 3-chlorophenyl, 2,5-dimethoxyphenyl, furyl, pyrollyl, thienyl, thiazolyl, imidazolyl, pyridyl, oxazolyl, indolyl, benzofuranyl or benzothienyl.

As a second embodiment the invention includes a cytotoxic agent consisting of a taxanes linked to a cell binding agents consisting of the compounds of the following formula (III) 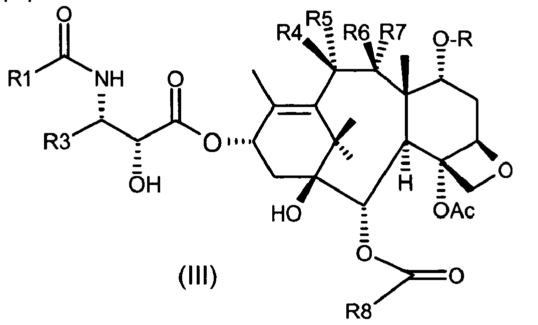 wherein:
**R**_{**1**}or R₃ or R₄ is a linker
**R**_{**2**} is an alkyl radical having from 1 to 10 carbon atoms, alkenyl or alkynyl having from 2 to 10 carbon atoms, cycloalkyl or cycloalkenyl having from 3 to 10 carbon atoms, optionally substituted aryl or heterocyclic radical.
**R**_{**3**} is an optionally substituted aryl or heterocyclic radical, alkyl having from 1 to 10 carbon atoms, alkenyl or alkynyl having from 2 to 10 carbon atoms, cycloalkyl or cycloalkenyl having from 3 to 10 carbon atoms
**R**_{**4**} is an H, an hydroxy radical, an alkoxy, an alkenyloxy, an optionally substituted alkanoyloxy, aroyloxy, alkenoyloxy, alkynoyloxy, cycloalkanoyloxy, alkoxyacetyl, alkylthioacetyl, alkyloxycarbonyloxy, cycloalkyloxy, cycloalkenyloxy, carbamoyloxy, alkylcarbamoyloxy, or dialkylcarbamoyloxy, a heterocyclic or aryl ether, ester or carbamate, or, a linear, branched, or cyclic alkyl or alkenyl ester or ether having from 1 to 10 carbon atoms or a carbamate of the formula -OCOX,
   wherein X is a nitrogen-containing heterocycle such as unsubstituted or substituted piperidino, morpholino, piperazino, N-methylpiperazino, or a carbamate of the formula -OCON**R**_{**9**}**R**_{**10**}, wherein **R**_{**9**} and **R**_{**10**} are the same or different and are H, linear, branched, or cyclic alkyl having from 1 to 10 atoms or simple or substituted aryl having from 1 to 10 carbon atoms.
**R**_{**5**} or **R**_{**7**} is H
**R**_{**6**} is a H
**R**_{**7**} or **R**_{**5**} and **R** form a bond (cyclic ether)
**R**_{**8**} = optionally substituted aryl or heterocyclic radical

The cell binding agent covently linked to the taxane is selected from the group consisting of antibodies, an antibody fragment, interferons, lymphokines, hormones, vitamins, growth factors, colony stimulating factors, and transferring, more preferably the cell binding agent is an antibody, and more particularly a monoclonal antibody.

The the cell binding agent can also be choosen among an antigen specific antibody fragment and more specifically among the antibody fragment sFV, Fab, Fab', or F(ab')₂. The cell binding agent may also be represented by a growth factor or acolony stimulating factor.

As a third embodiment the invention includes a therapeutic composition comprising:
(A) a therapeutically effective amount of a cytotoxic agent of formula (III) and
(B) a pharmaceutically acceptable carrier.

As a fourth embodiment the invention includes method of killing selected cell populations comprising contacting target cells or tissue containing target cells with an effective amount of the cytotoxic agent previously described.

The present invention will be more completely described in the following examples which have not to be considered as a limitation of the invention.

The following compounds of the invention have been synthesized

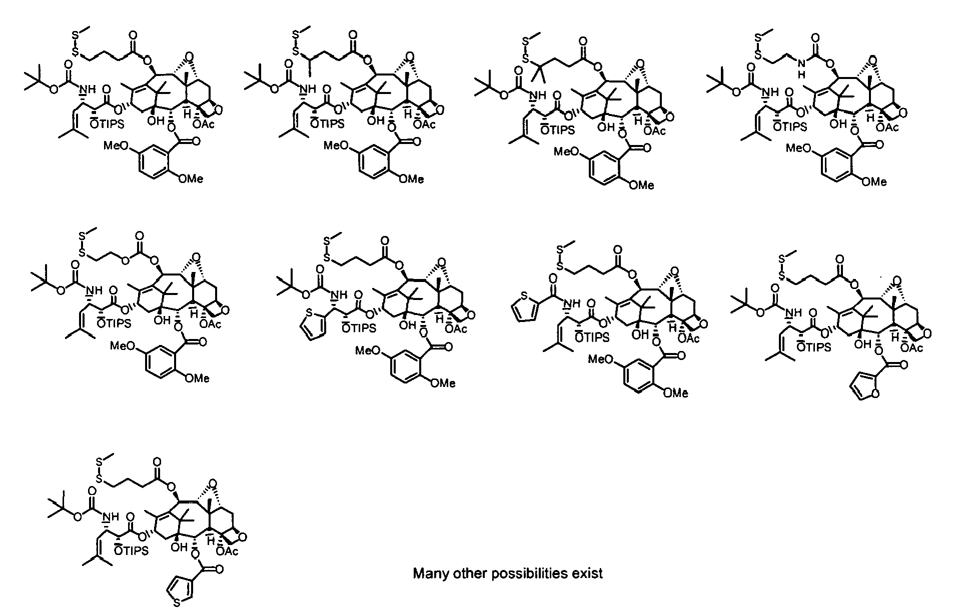

### 3'-isobutenyl series

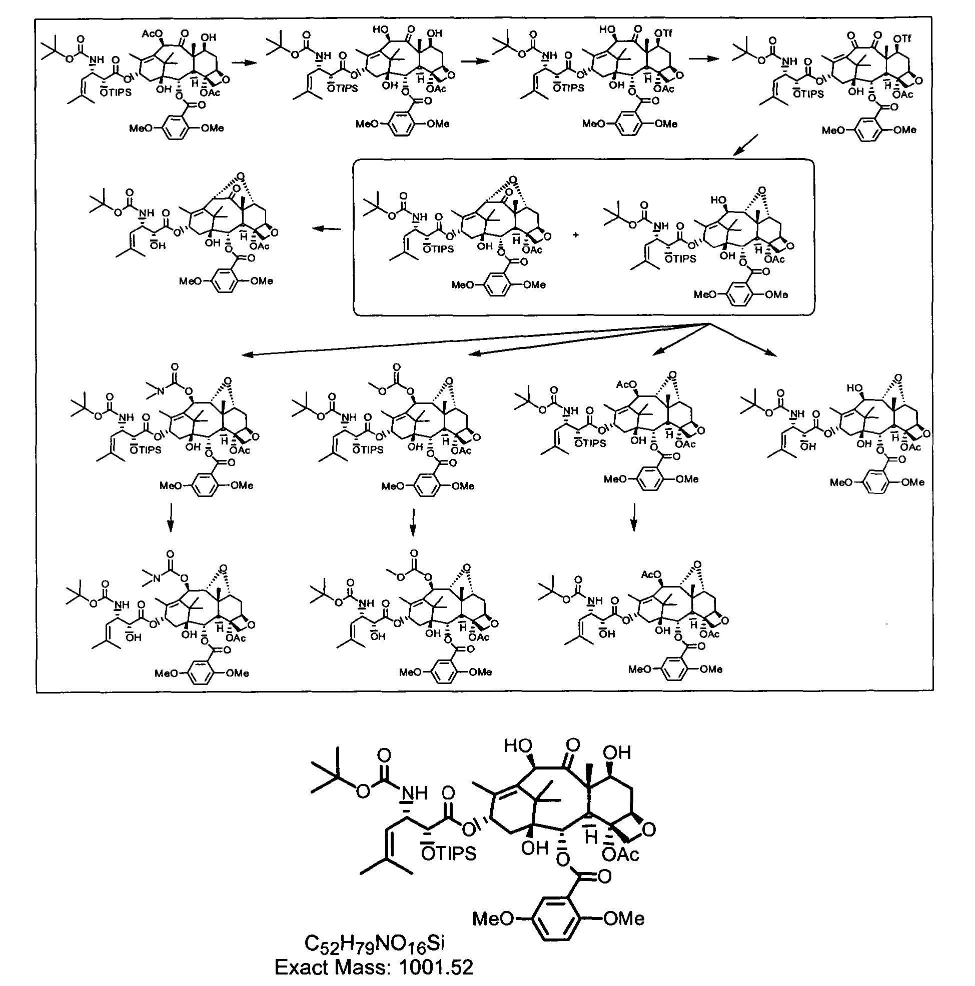

### 2'-(triisopropylsilyloxy)-3'-dephenyl-3'-(isobutenyl)-2-debenzoyl-2-(2,5-dimethoxybenzoyl)-docetaxel.

To a solution of 10-acetoxy-2'-(triisopropylsilyloxy)-3'-dephenyl-3'-(isobutenyl)-2-debenzoyl-2-(2,5-dimethoxybenzoyl)-docetaxel (1.30 g, 1.25 mmol) in ethanol (25 mL) was added hydrazine monohydrate (10.5 mL) with stirring. After 15 minutes the reaction was diluted with ethyl acetate (50 mL) and the organic layer was extracted with ammonium chloride (50 mL), water (50 mL), and brine (50 mL). The organic layer was then dried over anhydrous sodium sulfate, filtered, and concentrated in vacuo. The residue was purified on a silica gel column using 40 % ethyl acetate in hexane as the eluant. The fractions containing the desired product were pooled and concentrated to give 1.1 g of 2'-(triisopropylsilyloxy)-3'-dephenyl-3'-(isobutenyl)-2-debenzoyl-2-(2,5-dimethoxybenzoyl)-docetaxel as a white solid. ¹H NMR (CDCl₃) δ 1.08 (s, 24 H), 1.23 (s, 3H), 1.36 (s, 9 H), 1.67 (s, 3 H), 1.70 (s, 3 H), 1.76 (s, 3 H), 1.82 (m, 1 H), 1.88 (s, 3 H), 2.16 (s, 3 H), 2.31 (m, 1 H), 2.50 (m, 2 H), 3.17 (br s, 1 H), 3.79 (s, 3 H), 3.85 (d, *J* = 6.4 Hz, 1 H), 3.95 (s, 1 H), 4.18 (m, 2 H), 4.29 (d, *J* = 8.4 Hz, 1 H), 4.37 (d, *J* = 2 Hz, 1 H), 4.41 (d, *J* = 8.4 Hz, 1 H), 4.74 (t, *J* = 9 Hz, 1 H), 4.90 (t, *J* = 9.8 Hz, 2 H), 5.17 (d, *J* = 1.6 Hz, 1 H), 5.32 (d, *J* = 9.2 Hz, 1 H), 5.65 (d, *J* = 6.8 Hz, 1 H), 6.10 (t, *J* = 8.8 Hz, 1 H), 6.93 (d, *J* = 9.2 Hz, 1 H), 7.05 (dd, *J* = 9.2, 3.0 Hz, 1 H), 7.28 (d, *J* = 3.0 Hz, 1 H). m/z LC/MS for C₅₂H₇₉NO₁₆SiNa⁺: calcd: 1024.5; found: 1024.3.

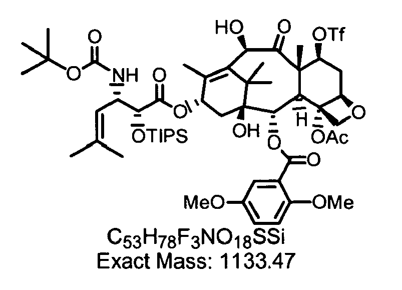

### 7-(trifluoromethanesulfonyloxy)-2'-(triisopropylsilyloxy)-3'-dephenyl-3'-(isobutenyl)-2-debenzoyl-2-(2,5-dimethoxybenzoyl)-docetaxel

A solution of 2'-(triisopropylsilyloxy)-3'-dephenyl-3'-(isobutenyl)-2-debenzoyl-2-(2,5-dimethoxy-benzoyl)-docetaxel (1.1 g, 1.1 mmol) in methylene chloride (7 mL) and pyridine (0.44 mL, 5.5 mmol) was cooled to -30°C in a dry ice and acetone bath. A solution containing triflic anhydride (0.37 mL, 2.2 mmol) dissolved in methylene chloride (0.3 mL) was added dropwise. The resulting solution was allowed to gradually warm to room temperature with stirring. After one hour the reaction was diluted with ethyl acetate (25 mL) and the organic layer was extracted with ammonium chloride (25 mL), water (25 mL), and brine (25 mL). The organic layer was then dried over anhydrous sodium sulfate, filtered, and concentrated in vacuo. The residue was purified on a silica gel column using 20 % ethyl acetate in hexane as the eluant. Fractions containing the desired product were pooled and concentrated to give 565 mg of 7-(trifluoromethanesulfonyloxy)-2'-(triisopropylsilyloxy)-3'-dephenyl-3'-(isobutenyl)-2-debenzoyl-2-(2,5-dimethoxy-benzoyl)-docetaxel as a solid.
¹H NMR (CDCl₃) δ 1.10 (m, 24 H), 1.26 (s, 3 H), 1.38 (s, 9 H), 1.68 (s, 3 H), 1.72 (s, 3 H), 1.92 (s, 3 H), 1.95 (s, 3 H), 2.20 (s, 3 H), 2.28 (m, 1 H), 2.31 (m, 1 H), 2.50 (m, 1 H), 2.80 (m, 1 H), 3.80 (s, 3 H), 3.95 (d, *J* = 6.4 Hz, 1 H), 3.98 (s, 3 H), 4.36 (d, *J* = 8.0 Hz, 1 H), 4.38 (d, *J* = 2.4 Hz, 1 H), 4.44 (d, *J* = 8.0 Hz, 1 H), 4.76 (t, *J* = 9.2 Hz, 1 H), 4.88 (m, 2 H), 5.33 (m, 2 H), 5.38 (dd, *J* = 6.8, 10.4 Hz, 1 H), 5.68 (d, *J* = 6.4 Hz, 1 H), 6.13 (t, *J* = 8.8 Hz, 1 H), 6.95 (d, *J* = 9.2 Hz, 1 H), 7.07 (dd, *J* = 3.2, 9.2 Hz, 1 H), 7.26 (d, *J* = 3.2 Hz, 1 H). m/z LC/MS for C₅₃H₇₈F₃NO₁₈SSiNa⁺:
calcd: 1156.4; found: 1156.1.

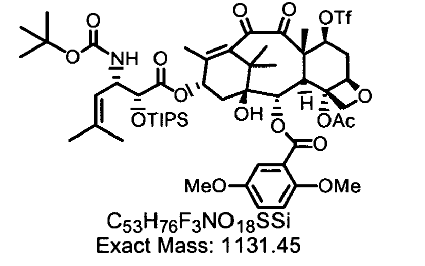

### 7-(trifluoromethanesulfonyloxy)-2'-(triisopropylsilyloxy)-3'-dephenyl-3'-(isobutenyl)-10-oxo-2-debenzoyl-2-(2,5-dimethoxybenzoyl)-docetaxel

To a stirred solution of 7-(trifluoromethanesulfonyloxy)-2'-(triisopropylsilyloxy)-3'-dephenyl-3'-(isobutenyl)-2-debenzoyl-2-(2,5-dimethoxybenzoyl)-docetaxel (565 mg, 0.5 mmol) in methylene chloride (20 mL) was added 4-methylmorpholine N-oxide (NMO) (117 mg, 1.00 mmol) followed by the addition of tetrapropylammonium perruthenate (TPAP) (10 mg, 0.03 mmol). The resulting mixture was allowed to stir at room temperature with monitoring. After 8 hours the reaction was filtered through celite and the pad of celite was rinsed with methylene chloride. The combined supernatant was concentrated in vacuo and the resulting residue was purified on a silica gel column using 25 % ethyl acetate in hexane. The fractions containing the product were pooled and concentrated to give 325 mg of 7-(trifluoromethanesulfonyloxy)-2'-(triisopropylsilyloxy)-3'-dephenyl-3'-(isobutenyl)-10-oxo-2-debenzoyl-2-(2,5-dimethoxybenzoyl)-docetaxel as a solid.
¹H NMR (CDCl₃) δ 1.10 (m, 21 H), 1.20 (s, 3 H), 1.30 (s, 3 H), 1.38 (s, 9 H), 1.68 (s, 3 H), 1.72 (s, 3 H), 1.92 (s, 3 H), 1.94 (s, 3 H), 2.18 (s, 3 H), 2.23 (m, 1 H), 2.35 (m, 1 H), 2.63 (m, 1 H), 2.83 (m, 1 H), 3.71 (m, 2 H), 3.81 (s, 3 H), 4.00 (s, 3 H), 4.34 (d, *J* = 8.4 Hz, 1 H), 4.39 (d, *J* = 2.0 Hz, 1 H), 4.45 (d, *J* = 8.4 Hz, 1 H), 4.78 (t, J = 9.2 Hz, 1 H), 4.87 (m, 2 H), 5.19 (dd, *J* = 8.0, 10.0 Hz, 1 H), 5.34 (d, *J* = 8.8 Hz, 1 H), 5.79 (d, *J* = 6.0 Hz, 1 H), 6.09 (t, *J* = 8.8 Hz, 1 H), 6.98 (d, *J* = 9.2 Hz, 1 H), 7.09 (dd, *J* = 3.2 9.2 Hz, 1 H), 7.25 (d, *J* = 3.2 Hz, 1 H). m/z LC/MS for C₅₃H₇₆F₃NO₁₈SSiNa⁺: calcd:1154.4; found: 1154.4.

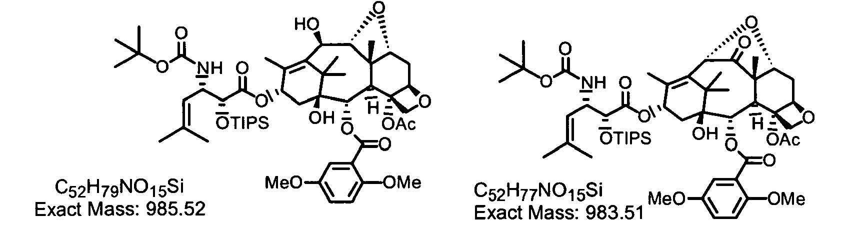

### 7α,9α-epoxy-2'-(triisopropylsilyloxy)-3'-dephenyl-3'-(isobutenyl)-2-debenzoyl-2-(2,5-dimethoxybenzoyl)-docetaxel

To a well stirred mixture of 7-(trifluoromethane-sulfonyloxy)-2'-(triisopropylsilyloxy)-3'-dephenyl-3'-(isobutenyl)-10-oxo-2-debenzoyl-2-(2,5-dimethoxybenzoyl)-docetaxel (323 mg, 0.286 mmol) in ethanol (3.5 mL) was added sodium borohydride (58 mg, 1.5 mmol). After 10 minutes the reaction was diluted with ethyl acetate (15 mL) and extracted twice with brine (15 mL). The organic layer was dried over anhydrous sodium sulfate, filtered, and concentrated in vacuo. The residue was purified by silica ptlc using 50 % ethyl acetate in hexane as the developing solvent to give the desired product, 7α,9α-epoxy-2'-(triisopropylsilyloxy)-3'-dephenyl-3'-(isobutenyl)-2-debenzoyl-2-(2,5-dimethoxybenzoyl)-docetaxel (171 mg) and the side product 7α,10α-epoxy-2'-(triisopropylsilyloxy)-3'-dephenyl-3'-(isobutenyl)-2-debenzoyl-2-(2,5-dimethoxybenzoyl)-docetaxel (78 mg).

### 7α,9α-epoxy-2'-(triisopropylsilyloxy)-3'-dephenyl-3'-(isobutenyl)-2-debenzoyl-2-(2,5-dimethoxybenzoyl)-docetaxel:

¹H NMR (CDCl₃) δ 1.09 (m, 24 H), 1.24 (s, 3 H), 1.38 (s, 9 H), 1.68 (s, 3 H), 1.71 (s, 3 H), 1.78 (s, 3 H), 1.97 (s, 3 H), 2.10 (s, 3 H), 2.23 - 2.33 (m, 2 H), 2.34 - 2.45 (m, 2 H), 2.50 (d, *J* = 3.2 Hz, 1 H), 3.80 (s, 3 H), 3.98 (s, 3 H), 4.02 (d, *J* = 6.0 Hz, 1 H), 4.25 (d, *J* = 7.2 Hz, 1 H), 4.34 (d, *J* = 7.2 Hz, 1 H), 4.45 (d, *J* = 2.4 Hz, 1 H), 4.73 - 4.83 (m, 4 H), 4.98 (m, 2 H), 5.35 (d, *J* = 8.8 Hz, 1 H), 5.67 (d, *J* = 6.4 Hz, 1 H), 5.97 (t, *J* = 8.8 Hz, 1 H), 6.96 (d, *J* = 9.2 Hz, 1 H), 7.06 (dd, *J* = 3.2 9.2 Hz, 1 H), 7.29 (d, *J* = 3.2 Hz, 1 H). m/z LC/MS for C₅₂H₇₉NO₁₅SiNa⁺: calcd: 1008.5; found: 1008.4.

### 7α,10α-epoxy-2'-(triisopropylsilyloxy)-3'-dephenyl-3'-(isobutenyl)-2-debenzoyl-2-(2,5-dimethoxybenzoyl)-docetaxel:

¹H NMR (CDCl₃) δ 1.10 (m, 21 H), 1.19 (s, 3 H), 1.34 (s, 3 H), 1.39 (s, 9 H), 1.66 (s, 3 H), 1.70 (s, 3 H), 1.84 (s, 3 H), 1.88 (s, 3 H), 2.07 (s, 3 H), 2.21 (m, 1 H), 2.29 (m, 1 H), 2.38 (m, 2 H), 3.66 (d,*J*= 7.6 Hz, 1 H), 3.81 (s, 3 H), 3.96 (s, 3 H), 4.39 - 4.47 (m, 4 H), 4.74 (t, *J* = 8.4 Hz, 1 H), 4.82 (br s, 1 H), 4.90 (m, 1 H), 5.08 (d, *J* = 4.4 Hz, 1 H), 5.35 (d, *J* = 8.8 Hz, 1 H), 5.42 (d, *J* = 6.0 Hz, 1 H), 6.12 (t, *J* = 8.8 Hz, 1 H), 6.95 (d, *J* = 8.8 Hz, 1 H), 7.07 (dd, *J* = 3.2, 8.8 Hz, 1 H), 7.32 (d, *J* = 3.2 Hz, 1 H). m/z LC/MS for C₅₂H₇₇NO₁₅SiNa⁺: calcd: 1006.5; found: 1006.4.

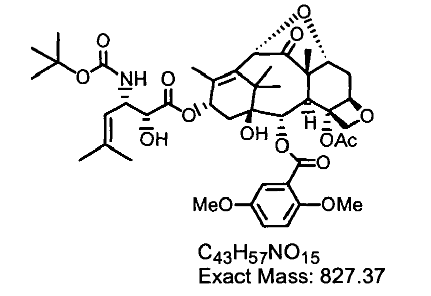

### 7α,10α-epoxy-3'-dephenyl-3'-(isobutenyl)-2-debenzoyl-2-(2,5-dimethoxybenzoyl)-docetaxel

A solution containing 7α,10α-epoxy-2'-(triisopropylsilyloxy)-3'-dephenyl-3'-(isobutenyl)-2-debenzoyl-2-(2,5-dimethoxy-benzoyl)-docetaxel (20 mg, 0.02 mmol) dissolved in a 1:1 mixture of acetonitrile-pyridine (2 mL) was cooled to 0°C in an ice bath. To this was added hydrogen fluoride-pyridine (0.2 mL) and the solution was allowed to gradually warm to room temperature. After 16 hours the reaction was diluted with ethyl acetate (15 mL) and quenched with a saturated sodium bicarbonate solution (15 mL). The organic layer was washed once with brine (20 mL) and then dried over anhydrous sodium sulfate, filtered, and concentrated in vacuo. The crude residue was purified by silica ptlc using 70 % ethyl acetate in hexane as the developing solvent. The band containing the desired product was scraped and rinsed with ethyl acetate at the filter. The organic layers were combined and concentrated in vacuo to give 12 mg of 7α,10α-epoxy-3'-dephenyl-3'-(isobutenyl)-2-debenzoyl-2-(2,5-dimethoxybenzoyl)-docetaxel as a white solid.
¹H NMR (CDCl₃) δ 1.19 (s, 3 H), 1.32 (s, 3 H), 1.41 (s, 9 H), 1.73 (s, 6 H), 1.85 (s, 3 H), 1.89 (s, 3 H), 2.03 (s, 3 H), 2.28 - 2.42 (m, 4 H), 3.69 (dd, *J* = 2.0, 7.2 Hz, 1 H), 3.81 (s, 3 H), 3.89 (s, 3 H), 4.25 (dd, *J* = 2.4, 4.4, 1 H), 4.34 (d, *J* = 6.0 Hz, 1 H), 4.36 (d, *J* = 6.0 Hz, 1 H), 4.53 (d, *J* = 7.6 Hz, 1 H), 4.82 (m, 2 H), 5.00 (d, *J* = 9.2 Hz, 1 H), 5.08 (m, 1 H), 5.25 (d, *J* = 8.8 Hz, 1 H), 5.35 (d, *J* = 6.0 Hz, 1 H), 6.17 (t, *J* = 7.6 Hz, 1 H), 6.94 (d, *J* = 9.2 Hz, 1 H), 7.06 (dd, *J* = 3.2, 9.2 Hz, 1 H), 7.28 (d, *J* = 3.2 Hz, 1 H). m/z LC/MS for C₄₃H₅₇NO₁₅Na⁺: calcd: 850.4; found: 850.3.

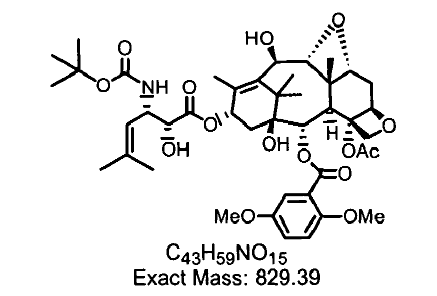

### 7α,9α-epoxy-2'-3'-dephenyl-3'-(isobutenyl)-2-debenzoyl-2-(2,5-dimethoxybenzoyl)-docetaxel

A solution containing 7α,9α-epoxy-2'-(triisopropylsilyloxy)-3'-dephenyl-3'-(isobutenyl)-2-debenzoyl-2-(2,5-dimethoxybenzoyl)-docetaxel (25 mg, 0.025 mmol) dissolved in a 1:1 mixture of acetonitrile:pyridine (2 mL) was cooled to 0°C in an ice bath. To this was added hydrogen fluoride-pyridine (0.25 mL) and the solution was allowed to gradually warm to room temperature. After 16 hours the reaction was diluted with ethyl acetate (15 mL) and quenched with a saturated sodium bicarbonate solution (15 mL). The organic layer was washed once with brine (20 mL) and then dried over anhydrous sodium sulfate, filtered, and concentrated in vacuo. The crude residue was purified by silica ptlc using 70 % ethyl acetate in hexane as the developing solvent. The band containing the desired product was scraped and rinsed with ethyl acetate at the filter. The organic layers were combined and concentrated in vacuo to give 12 mg of 7α,9a-epoxy-3'-dephenyl-3'-(isobutenyl)-2-debenzoyl-2-(2,5-dimethoxybenzoyl)-docetaxel as a white solid.

¹H NMR (CDCl₃) δ 1.12 (s, 3 H), 1.23 (s, 3 H), 1.41 (s, 9 H), 1.73 (s, 6 H), 1.80 (s, 3 H), 2.01 (s, 3 H), 2.07 (s, 3 H), 2.15 - 2.21 (m, 1 H), 2.23 - 2.28 (m, 1 H), 2.36 - 2.44 (m, 1 H), 2.43 - 2.49 (m, 1 H), 2.52 (d, *J* = 6.8 Hz, 1 H), 3.75 (br s , 1 H), 3.80 (s, 3 H), 3.93 (s, 3 H), 4.05 (d, *J* = 6.4 Hz, 1 H), 4.26 (d, *J* = 2.4, 4.8 Hz, 1 H), 4.30 (d, *J* = 7.6 Hz, 1 H), 4.32 (d, *J* = 7.6 Hz, 1 H), 4.76 - 4.83 (m, 4 H), 4.91 (t, *J* = 2.0 Hz, 1 H), 4.98 (d, *J* = 9.6 Hz, 1 H), 5.26 (d, *J* = 8.4 Hz, 1 H), 5.65 (d, *J* = 6.4 Hz, 1 H), 6.01 (t, *J* = 8.8 Hz, 1 H), 6.95 (d, *J* = 9.2 Hz, 1 H), 7.06 (dd, *J* = 3.2 9.2 Hz, 1 H), 7.28 (d, *J* = 3.2 Hz, 1 H). m/z LC/MS for C₄₃H₅₉NO₁₅Na⁺: calcd: 852.4; found: 852.3.

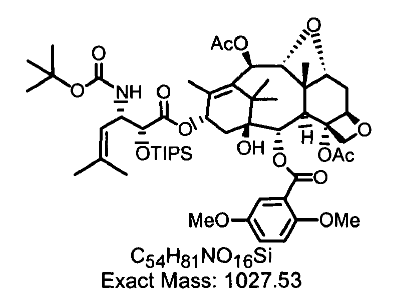

### 7α,9α-epoxy-2'-(triisopropylsilyloxy)-3'-dephenyl-3'-(isobutenyl)-2-debenzoyl-2-(2,5-dimethoxybenzoyl)-10-acetoxy-docetaxel

To a solution of 7α,9α-epoxy-2'-(triisopropylsilyloxy)-3'-dephenyl-3'-(isobutenyl)-2-debenzoyl-2-(2,5-dimethoxybenzoyl)-docetaxel (85 mg, 0.086 mmol) dissolved in pyridine (1 mL) under nitrogen was added dimethylamino pyridine (DMAP) (16 mg, 0.13 mmol) and acetic anhydride (0.025 mL, 0.26 mmol). The reaction was stirred at room temperature and after 4 hours was diluted with ethyl acetate (20 mL) and washed once with brine (25 mL). The organic layer was separated, dried over anhydrous sodium sulfate, filtered, and concentrated in vacuo. The residue was purified by silica ptlc using 50 % ethyl acetate in hexane as the developing solvent. The band containing the desired product was scraped and rinsed with ethyl acetate at the filter. The organic layers were combined and concentrated in vacuo to give 75 mg of 7α,9α-epoxy-2'-(triisopropylsilyloxy)-3'-dephenyl-3'-(isobutenyl)- 2-debenzoyl-2-(2,5-dimethoxybenzoyl)-10-acetoxy-docetaxel as a white solid.
¹H NMR (CDCl₃) δ 1.10 (m, 21 H), 1.22 (s, 6 H), 1.38 (s, 9 H), 1.67 (s, 3 H), 1.70 (s, 3 H), 1.73 (s, 3 H), 1.94 (s, 3 H), 2.07 (s, 3 H), 2.10 (s, 3 H), 2.31 (m, 3 H), 2.48 (m, 1 H), 3.78 (s, 3 H), 3.97 (s, 3 H), 3.98 (d, *J* = 6.0 Hz, 1 H), 4.24 (d, *J* = 7.2 Hz, 1 H), 4.32 (d, *J* = 7.2 Hz, 1 H), 4.45 (d, *J* = 2.0 Hz, 1 H), 4.77 (m, 2 H), 4.82 (d, *J* = 6.0 Hz, 1 H), 4.91 (s, 1 H), 4.93 (d, *J* = 9.2 Hz, 1 H), 5.36 (d, *J* = 8.4 Hz, 1 H), 5.68 (d, *J* = 9.2 Hz, 1 H), 5.70 (d, *J* = 6.0 Hz, 1 H), 5.89 (t, *J* = 8.8 Hz, 1 H), 6.94 (d, *J* = 9.2 Hz, 1 H), 7.05 (dd, *J* = 3.2 9.2 Hz, 1 H), 7.29 (d, *J* = 3.2 Hz, 1 H). m/z LC/MS for C₅₄H₈₁NO₁₆SiNa⁺: calcd: 1050.5; found: 1050.5.

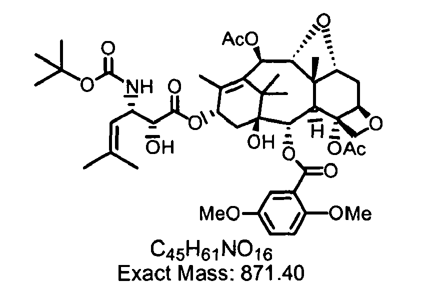

### 7α,9α-epoxy-3'-dephenyl-3'-(isobutenyl)-2-debenzoyl-2-(2,5-dimethoxybenzoyl)-10-acetoxy-docetaxel

A solution containing 7α,9α-epoxy-2'-(triisopropylsilyloxy)-3'-dephenyl-3'-(isobutenyl)-2-debenzoyl-2-(2,5-dimethoxybenzoyl)-10-acetoxy-docetaxel (75 mg, 0.073 mmol) dissolved in a 1:1 mixture of acetonitrile:pyridine (3 mL) was cooled to 0°C in an ice bath. To this was added hydrogen fluoride-pyridine (0.5 mL) and the solution was allowed to gradually warm to room temperature. After 16 hours the reaction was diluted with ethyl acetate (25 mL) and quenched with a saturated sodium bicarbonate solution (25 mL). The organic layer was washed once with brine (30 mL) and then dried over anhydrous sodium sulfate, filtered, and concentrated in vacuo. The crude residue was purified by silica ptlc using 70 % ethyl acetate in hexane as the developing solvent. The band containing the desired product was scraped and rinsed with ethyl acetate at the filter. The organic layers were combined and concentrated in vacuo to give 52 mg of 7α,9α-epoxy-3'-dephenyl-3'-(isobutenyl)-2-debenzoyl-2-(2,5-dimethoxybenzoyl)-10-acetoxy-docetaxel.
¹H NMR (CDCl₃) δ 1.26 (s, 3 H), 1.28 (s, 3 H), 1.41 (s, 9 H), 1.74 (s, 6 H), 1.76 (s, 3 H), 1.99 (s, 3 H), 2.09 (s, 3 H), 2.11 (s, 3 H), 2.18 (m, 1 H), 2.27 (m, 1 H), 2.36 (m, 1 H), 2.49 (m, 1 H), 3.81 (s, 3 H), 3.91 (s, 1 H), 3.94 (s, 3 H), 4.02 (d,*J*= 6.0 Hz, 1 H), 4.28 (m, 2 H), 4.33 (d,*J*= 7.2 Hz, 1 H), 4.80 (m, 2 H), 4.86 (d,*J*= 6.4 Hz, 1 H), 4.91 (s, 1 H), 5.00 (d,*J*= 9.2 Hz, 1 H), 5.29 (d,*J*= 8.4 Hz, 1 H), 5.67 (d, *J* = 6.4 Hz, 1 H), 5.71 (d, *J* = 6.4 Hz, 1 H), 6.01 (t, *J* = 8.8 Hz, 1 H), 6.96 (d, *J* = 9.2 Hz, 1 H), 7.07 (dd, *J* = 3.2 9.2 Hz, 1 H), 7.28 (d, *J* = 3.2 Hz, 1 H). m/z LC/MS for C₄₅H₆₁NO₁₆Na⁺: calcd: 894.4; found: 894.5.

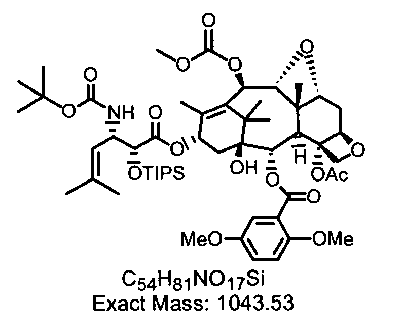

### 7α,9α-epoxy-2'-(triisopropylsilyloxy)-3'-dephenyl-3'-(isobutenyl)-2-debenzoyl-2-(2,5-dimethoxybenzoyl)-10-methoxycarbonyloxy-docetaxel

A solution of 7α,9α-epoxy-2'-(triisopropylsilyloxy)-3'-dephenyl-3'-(isobutenyl)-2-debenzoyl-2-(2,5-dimethoxybenzoyl)-docetaxel (28 mg, 0.029 mmol) dissolved in anhydrous tetrahydrofuran (0.65 mL) under nitrogen was cooled to -40°C in a dry ice and acetone bath. To this solution was added 1.0 M LiHMDS (0.04 mL, 0.039 mmol) and allowed to stir for 15 minutes. To this was added methyl chloroformate (0.003 mL, 0.036 mmol) and the reaction was monitored at -40°C. After one hour the reaction was diluted with ethyl acetate (15 mL) and extracted with water (15 mL) followed by washing the organic layer with brine (15 mL). The organic layer was dried over anhydrous sodium sulfate, filtered, and concentrated in vacuo. The residue was purified by silica ptlc using 5 % methanol in methylene chloride as the developing solvent. The band containing the desired product was scraped and rinsed with ethyl acetate at the filter. The organic layers were combined and concentrated in vacuo to give 9 mg of 7α,9α-epoxy-2'-(triisopropylsilyloxy)-3'-dephenyl-3'-(isobutenyl)-2-debenzoyl-2-(2,5-dimethoxybenzoyl)-10-methoxycarbonyloxy-docetaxel as a white solid.
¹H NMR (CDCl₃) δ 1.10 (m, 21 H), 1.22 (s, 3 H), 1.24 (s, 3 H), 1.39 (s, 9 H), 1.68 (s, 3 H), 1.71 (s, 3 H), 1.75 (s, 3 H), 1.96 (s, 3 H), 2.08 (s, 3 H), 2.30 (m, 3 H), 2.50 (m, 1 H), 3.78 (s, 3 H), 3.80 (s, 3 H), 3.98 (d,*J*= 6.0 Hz, 1 H), 3.99 (s, 3 H), 4.25 (d,*J*= 7.2 Hz, 1 H), 4.34 (d,*J*= 7.2 Hz, 1 H), 4.46 (d,*J*= 2.0 Hz, 1 H), 4.78 (m, 2 H), 4.91 (m, 3 H), 5.37 (d,*J*= 8.4 Hz, 1 H), 5.57 (d,*J*= 6.4 Hz, 1 H), 5.70 (d,*J*= 6.0 Hz, 1 H), 5.93 (t,*J*= 8.8 Hz, 1 H), 6.95 (d,*J*= 9.2 Hz, 1 H), 7.06 (dd,*J*= 3.2 9.2 Hz, 1 H), 7.30 (d,*J*= 3.2 Hz, 1 H). m/z LC/MS for C₄₅H₈₁NO₁₇SiNa⁺: calcd: 1066.5; found: 1066.3.

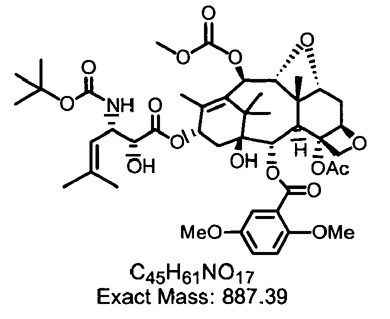

### 7α,9α-epoxy-3'-dephenyl-3'-(isobutenyl)-2-debenzoyl-2-(2,5-dimethoxybenzoyl)-10-methoxycarbonyloxy-docetaxel

A solution containing 7α,9α-epoxy-2'-(triisopropylsilyloxy)-3'-dephenyl-3'-(isobutenyl)-2-debenzoyl-2-(2,5-dimethoxybenzoyl)-10-methoxycarbonyloxy-docetaxel (9 mg, 0.008 mmol) dissolved in a 1:1 mixture of acetonitrile:pyridine (1.2 mL) was cooled to 0°C in an ice bath. To this was added hydrogen fluoride-pyridine (0.15 mL) and the solution was allowed to gradually warm to room temperature. After 16 hours the reaction was diluted with ethyl acetate (15 mL) and quenched with a saturated sodium bicarbonate solution (15 mL). The organic layer was washed once with brine (15 mL) and then dried over anhydrous sodium sulfate, filtered, and concentrated in vacuo. The crude residue was purified by silica ptlc using 60 % ethyl acetate in hexane as the developing solvent. The band containing the desired product was scraped and rinsed with ethyl acetate at the filter. The organic layers were combined and concentrated in vacuo to give 4 mg of 7α,9α-epoxy-3'-dephenyl-3'-(isobutenyl)-2-debenzoyl-2-(2,5-dimethoxybenzoyl)-10-methoxycarbonyloxy-docetaxel.
¹H NMR (CDCl₃) δ 1.25 (s, 3 H), 1.27 (s, 3 H), 1.40 (s, 9 H), 1.73 (s, 6 H), 1.76 (s, 3 H), 1.99 (s, 3 H), 2.07 (s, 3 H), 2.18 (m, 1 H), 2.25 (m, 1 H), 2.34 (m, 1 H), 2.48 (m, 1 H), 3.78 (s, 3 H), 3.80 (s, 3 H), 3.83 (s, 1 H), 3.93 (s, 3 H), 4.01 (d, *J* = 6.0 Hz, 1 H), 4.28 (m, 2 H), 4.31 (d, *J* = 7.6 Hz, 1 H), 4.80 (m, 2 H), 4.90 (s, 1 H), 4.94 (d, *J* = 7.6 Hz, 1 H), 4.97 (d, *J* = 9.6 Hz, 1 H), 5.27 (d, *J* = 7.6 Hz, 1 H), 5.55 (d, *J* = 6.0 Hz, 1 H), 5.70 (d, *J* = 6.0 Hz, 1 H), 6.03 (t, *J* = 8.8 Hz, 1 H), 6.95 (d, *J* = 9.2 Hz, 1 H), 7.06 (dd, *J* = 3.2 9.2 Hz, 1 H), 7.28 (d, *J* = 3.2 Hz, 1 H). m/z LC/MS for C₄₅H₆₁NO₁₇Na⁺: calcd: 910.4; found: 910.4.

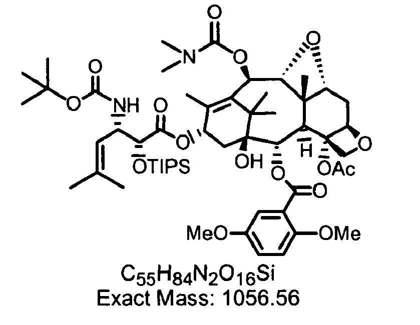

### 7α,9α-epoxy-2'-(triisopropylsilyloxy)-3'-dephenyl-3'-(isobutenyl)-2-debenzoyl-2-(2,5-dimethoxybenzoyl)-10-dimethyaminocarbonyloxy-docetaxel

A solution of 7α,9α-epoxy-2'-(triisopropylsilyloxy)-3'-dephenyl-3'-(isobutenyl)-2-debenzoyl-2-(2,5-dimethoxybenzoyl)-docetaxel (28 mg, 0.029 mmol) dissolved in anhydrous tetrahydrofuran (0.65 mL) under nitrogen was cooled to -40°C in a dry ice and acetone bath. To this solution was added 1.0 M LiHMDS (0.04 mL, 0.039 mmol) and allowed to stir for 15 minutes. To this was added dimethyl carbamyl chloride (0.003 mg, 0.031 mmol) and the reaction was monitored at -40°C. After one hour the reaction was diluted with ethyl acetate (15 mL) and extracted with water (15 mL) followed by washing the organic layer with brine (15 mL). The organic layer was dried over anhydrous sodium sulfate, filtered, and concentrated in vacuo. The residue was purified by silica ptlc using 5 % methanol in methylene chloride as the developing solvent. The band containing the desired product was scraped and rinsed with ethyl acetate at the filter. The organic layers were combined and concentrated in vacuo to give 20 mg of 7α,9α-epoxy-2'-(triisopropylsilyloxy)-3'-dephenyl-3'-(isobutenyl)-2-debenzoyl-2-(2,5-dimethoxybenzoyl)-10-dimethyamino-carbonyloxy-docetaxel as a white solid.

¹H NMR (CDCl₃) δ 1.10 (m, 21 H), 1.23 (s, 6 H), 1.39 (s, 9 H), 1.68 (s, 3 H), 1.71 (s, 3 H), 1.73 (s, 3 H), 1.98 (s, 3 H), 2.09 (s, 3 H), 2.31 (m, 3 H), 2.49 (m, 1 H), 2.93 m(s, 6 H), 3.80 (s, 3 H), 3.98 (s, 3 H), 3.96 (d, *J* = 6.0 Hz, 1 H), 4.25 (d, *J* = 7.2 Hz, 1 H), 4.33 (d, *J* = 7.2 Hz, 1 H), 4.46 (d, *J* = 2.0 Hz, 1 H), 4.77 (m, 2 H), 4.88 (d, *J* = 6.4 Hz, 1 H), 4.92 (m, 2 H), 5.36 (d, *J* = 8.4 Hz, 1 H), 5.68 (d, *J* = 6.0 Hz, 1 H), 5.71 (d, *J* = 6.4 Hz, 1 H), 5.93 (t, *J* = 8.8 Hz, 1 H), 6.95 (d, *J* = 9.2 Hz, 1 H), 7.05 (dd, *J* = 3.2 9.2 Hz, 1 H), 7.30 (d, *J* = 3.2 Hz, 1 H). m/z LC/MS for C₅₅H₈₄N₂O₁₆SiNa⁺:
calcd: 1079.6; found: 1079.5.

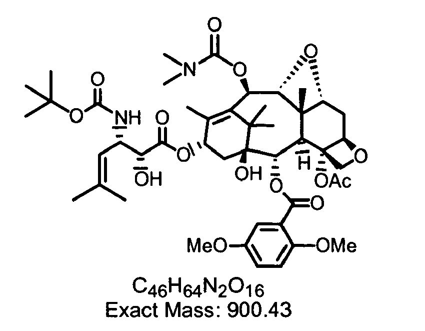

### 7α,9α-epoxy-3'-dephenyl-3'-(isobutenyl)-2-debenzoyl-2-(2,5-dimethoxybenzoyl)-10-dimethyaminocarbonyloxy-docetaxel

A solution containing 7α,9α-epoxy-2'-(triisopropylsilyloxy)-3'-dephenyl-3'-(isobutenyl)-2-debenzoyl-2-(2,5-dimethoxybenzoyl)-10-dimethyaminocarbonyloxydocetaxel (20 mg, 0.016 mmol) dissolved in a 1:1 mixture of acetonitrile:pyridine (1.2 mL) was cooled to 0°C in an ice bath. To this was added hydrogen fluoride-pyridine (0.25 mL) and the solution was allowed to gradually warm to room temperature. After 16 hours the reaction was diluted with ethyl acetate (15 mL) and quenched with a saturated sodium bicarbonate solution (15 mL). The organic layer was washed once with brine (15 mL) and then dried over anhydrous sodium sulfate, filtered, and concentrated in vacuo. The crude residue was purified by silica ptlc using 70 % ethyl acetate in hexane as the developing solvent. The band containing the desired product was scraped and rinsed with ethyl acetate at the filter. The organic layers were combined and concentrated in vacuo to give 12 mg of 7α,9α-epoxy-3'-dephenyl-3'-(isobutenyl)-2-debenzoyl-2-(2,5-dimethoxybenzoyl)-10-dimethyamino-carbonyloxy-docetaxel.
¹H NMR (CDCl₃) δ 1.24 (s, 3 H), 1.26 (s, 3 H), 1.40 (s, 9 H), 1.73 (s, 6 H), 1.74 (s, 3 H), 2.00 (s, 3 H), 2.07 (s, 3 H), 2.17 (m, 1 H), 2.25 (m, 1 H), 2.36 (m, 1 H), 2.48 (m, 1 H), 2.93 (s, 3 H), 2.94 (s, 3 H), 3.79 (s, 3 H), 3.86 (s, 1 H), 3.92 (s, 3 H), 4.02 (d,*J*= 6.0 Hz, 1 H), 4.28 (m, 2 H), 4.31 (d,*J*= 7.6 Hz, 1 H), 4.80 (m, 2 H), 4.89 (d,*J*= 6.0 Hz, 1 H), 4.90 (s, 1 H), 4.97 (d,*J*= 9.6 Hz, 1 H), 5.28 (d,*J*= 8.4 Hz, 1 H), 5.67 (d,*J*= 6.0 Hz, 1 H), 5.70 (d,*J*= 6.0 Hz, 1 H), 6.02 (t,*J*= 8.4 Hz, 1 H), 6.94 (d, J = 9.2 Hz, 1 H), 7.05 (dd,*J*= 3.2 9.2 Hz, 1 H), 7.29 (d,*J*= 3.2 Hz, 1 H). m/z LC/MS for C₄₆H₆₄N₂O₁₆Na⁺: calcd: 923.4; found: 923.4.

### 3'-(2-furyl) series

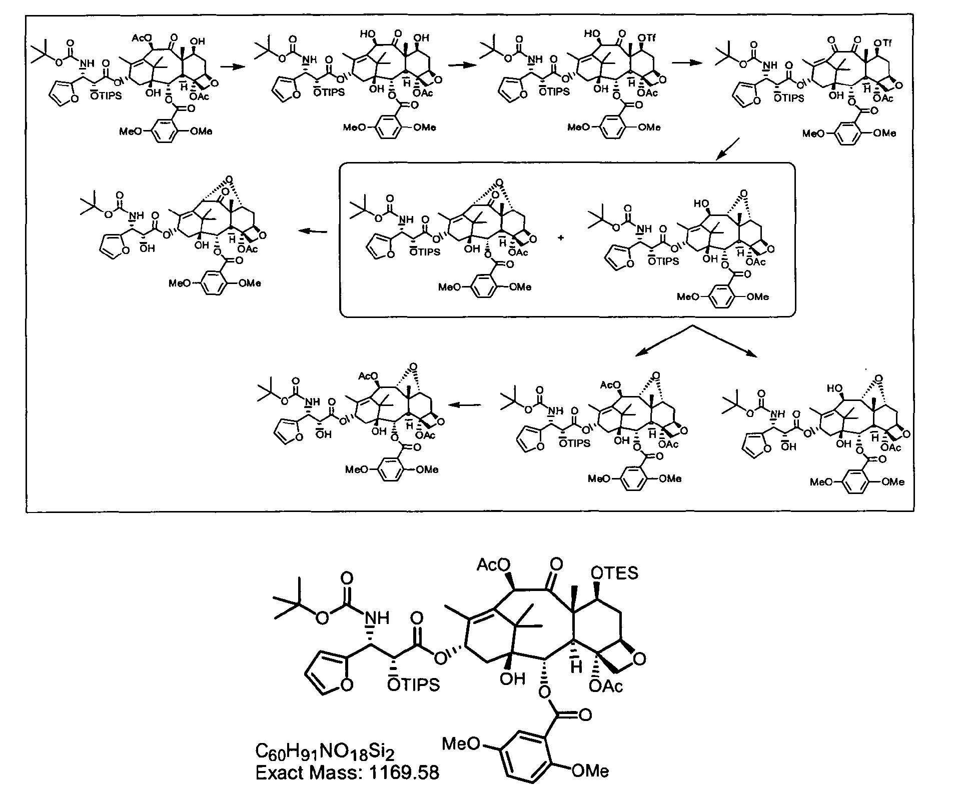

### 10-Acetoxy-7-(triethylsilyl)-2'-(triisopropylsilyloxy)-3'-dephenyl-3'-(2-furyl)-2-debenzoyl-2-(2,5-dimethoxybenzoyl)-docetaxel

A mixture of (3*R*,4*S*)-1-tert-butoxycarbonyl)-3-triisopropylsilyloxy-4-(2-furyl)-azetidin-2-one (1.27 g, 3.11 mmol) and 7-(triethylsilyl)-2-debenzoyl-2-(2,5-dimethoxybenzoyl)-baccatin III (1.9 g, 2.5 mmol) were dissolved in anhydrous THF (20 mL). The mixture was cooled to -40°C and 1.0 M LiHMDS (3.5 mL, 3.5 mmol) was added dropwise. The reaction was allowed to stir between -40 and -20°C for 1 hour, after which it was complete. The reaction was quenched with saturated aqueous ammonium chloride and extracted into ethyl acetate (100 mL x 2). The combined organic layers were washed with water (30 mL x 1), dried over magnesium sulfate and concentrated *in vacuo.* The crude residue was purified on a silica gel column with 30 % ethyl acetate in hexanes as the eluant, yielding 10-Acetoxy-7-(triethylsilyl)-2'-(triisopropylsilyloxy)-3'-dephenyl-3'-(2-furyl)-2-debenzoyl-2-(2,5-dimethoxybenzoyl)-docetaxel as a white solid (2.88 g, 98 %) :
¹H NMR (CDCl₃) δ 0.53 (m, 6 H), 0.89 (m, 30 H), 1.18 (s, 6 H), 1.36 (s, 9 H), 1.69 (s, 3 H), 1.85 (m, 1 H), 1.96 (s, 3 H), 2.11 (s, 3 H), 2.25 (s, 3 H), 2.35 (m , 2 H), 2.46 (m, 1 H), 3.33 (s, 1 H), 3.71 (s, 3 H), 3.72 (d, *J* = 6.8 Hz, 1 H), 3.87 (s, 3 H), 4.22 (d, *J* = 8.0 Hz, 1 H), 4.40 (m, 2 H), 4.84 (d, *J* = 8.0, 1 H), 4.89 (d, *J* = 1.2 Hz, 1 H), 5.24 (m , 2 H), 5.61 (d, *J* = 6.8 Hz, 1 H), 6.12 (t, *J* = 8.4 Hz, 1 H), 6.18 (d, *J* = 3.2 Hz, 1 H), 6.27 (dd, *J* = 3.2, 2.0 Hz, 1 H), 6.41 (s, 1 H), 6.88 (d, *J* = 9.2 Hz, 1 H), 6.97 (dd, *J* = 9.2, 3.2 Hz, 1 H), 7.20 (d, *J* = 3.2 Hz, 1 H), 7.28 (br s, 1 H). m/z LC/MS for C₆₀H₉₁NO₁₈Si₂Na⁺: calcd: 1192.6; found:1192.3.

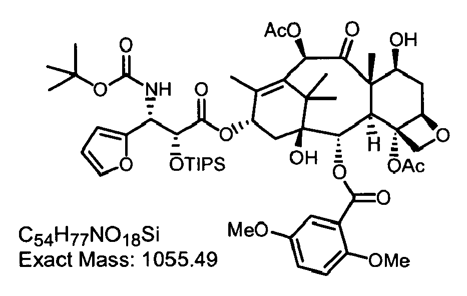

### 10-Acetoxy-2'-(triisopropylsilyloxy)-3'-dephenyl-3'-(2-furyl)-2-debenzoyl-2-(2,5-dimethoxybenzoyl)-docetaxel.

10-Acetoxy-7-(triethylsilyl)-2'-(triisopropylsilyloxy)-3'-dephenyl-3'-(2-furyl)-2-debenzoyl-2-(2,5-dimethoxybenzoyl)-docetaxel (670 mg, 0.57 mmol) was dissolved in ethanol (2.5 mL). A solution of 5 % hydrochloric acid in ethanol (5.0 mL) was added dropwise. The reaction was allowed to stir at room temperature for 5 hours, after which it was complete. The reaction was quenched with saturated aqueous sodium bicarbonate and the product was extracted into ethyl acetate (75 mL x 2). The combined ethyl acetate layers were washed with water (25 mL x 1) and brine (25 mL x 1), dried over magnesium sulfate and concentrated *in vacuo.* The crude 10-Acetoxy-2'-(triisopropylsilyloxy)-3'-dephenyl-3'-(2-furyl)-2-debenzoyl-2-(2,5-dimethoxybenzoyl)-docetaxel was used without purification :
¹H NMR (CDCl₃) δ 0.92 (m, 21 H), 1.12 (s, 3 H), 1.25 (s, 3 H), 1.39 (s, 9 H), 1.70 (s, 3 H), 1.85 (m, 4 H), 2.19 (s, 3 H), 2.27 (s, 3 H), 2.37 (m, 2 H), 2.51 (m, 1 H), 3.33 (s, 1 H), 3.74 (m, 4 H), 3.89 (s, 3 H), 4.26 (d, *J* = 8.4, 1 H), 4.38 (m, 2 H), 4.90 (m, 2 H), 5.24 (m, 2 H), 5.62 (d, *J* = 6.8 Hz, 1 H), 6.19 (m, 2 H), 6.30 (m, 2 H), 6.88 (d, *J* = 9.2, 1 H), 7.00 (dd, *J* = 9.2, 3.2 Hz, 1 H), 7.21 (d, *J* = 3.2 Hz, 1 H), 7.30 (br s, 1 H). m/z LC/MS for C₅₄H₇₇NO₁₈SiNa⁺: calcd: 1078.5; found:1078.3.

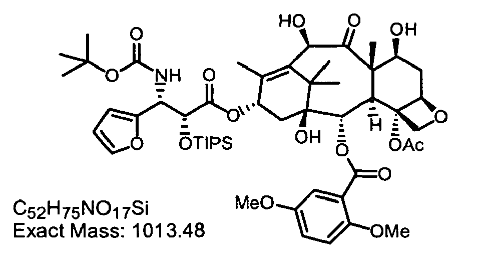

### 2'-(triisopropylsilyloxy)-3'-dephenyl-3'-(2-furyl)-2-debenzoyl-2-(2,5-dimethoxybenzoyl)-docetaxel

10-Acetoxy-2'-(triisopropylsilyloxy)-3'-dephenyl-3'-(2-furyl)-2-debenzoyl-2-(2,5-dimethoxybenzoyl)-docetaxel (605 mg, 0.573 mmol) was dissolved in ethanol (12.0 mL). Hydrazine monohydrate (5.0 mL) was added dropwise over 5 minutes, after which the reaction was complete. The reaction was diluted with ethyl acetate and quenched with saturated aqueous ammonium chloride. The product was extracted into ethyl acetate (75 mL x 2), washed with water (25 mL x 1) and brine (25 mL x 1), dried over anhydrous sodium sulfate and concentrated *in vacuo.* The crude residue was purified on a silica gel column with 40 % ethyl acetate in hexanes as the eluant, yielding 2'-(triisopropylsilyloxy)-3'-dephenyl-3'-(2-furyl)-2-debenzoyl-2-(2,5-dimethoxybenzoyl)-docetaxel as a white solid (507.6 mg, 87 %, two steps):
¹H NMR (CDCl₃) δ 0.95 (m, 21 H), 1.11 (s, 3 H), 1.24 (s, 3 H), 1.40 (s, 9 H), 1.79 (s, 3 H), 1.86 (m, 1 H), 1.91 (s, 3 H), 2.29 (s, 3 H), 2.36 (m, 2 H), 2.57 (m, 1 H), 3.29 (br s, 1 H), 3.76 (s, 3 H), 3.88 (d,*J*= 6.8 Hz, 1 H), 3.92 (s, 3 H), 4.19 (m, 2 H), 4.43 (d, *J* = 8.4 Hz, 1 H), 4.45 (d, *J* = 8.4 Hz, 1 H), 4.91 (m, 2 H), 5.24 (m, 3 H), 5.65 (d, *J* = 6.8 Hz, 1 H), 6.21 (m, 2 H), 6.31 (dd, *J* = 3.2, 1.6, 1 H), 6.90 (d, *J* = 9.2 Hz, 1 H), 7.02 (dd, *J* = 9.2, 3.2 Hz, 1 H), 7.23 (d, *J* = 3.2 Hz, 1 H), 7.30 (br s, 1 H). m/z LC/MS for C₅₂H₇₅NO₁₇SiNa⁺: calcd: 1036.5; found:1036.3.

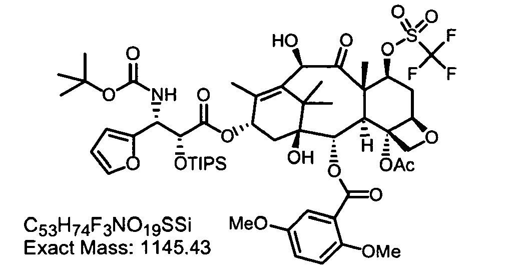

### 7-(trifluoromethanesulfonyloxy)-2'-(triisopropylsilyloxy)-3'-dephenyl-3'-(2-furyl)-2-debenzoyl-2-(2,5-dimethoxybenzoyl)-docetaxel

2'-(triisopropylsilyloxy)-3'-dephenyl-3'-(2-furyl)-2-debenzoyl-2-(2,5-dimethoxybenzoyl)-docetaxel (1.7 g, 1.68 mmol) was dissolved in methylene chloride (10.0 mL) and cooled to -30°C. Pyridine (0.68 mL, 8.4 mmol) was added, followed by triflic anhydride (0.57 mL, 3.4 mmol) in methylene chloride (0.5 mL), turning the reaction yellow. The reaction was allowed to warm to 0°C slowly over 1 hour, at which point it was complete. The product was extracted into ethyl acetate (150 mL x 1), washed with water (50 mL x 1) and brine (50 mL x 1), dried over magnesium sulfate and concentrated *in vacuo.* The crude reside was purified on a silica gel column with 25 % ethyl acetate in hexanes as the eluant, yielding 7-(trifluoromethanesulfonyloxy)-2'-(triisopropylsilyloxy)-3'-dephenyl-3'-(2-furyl)-2-debenzoyl-2-(2, 5-dimethoxybenzoyl)-docetaxel as a white solid(1.44 g, 75 %):
¹H NMR (CDCl₃) δ 0.94 (m, 21 H), 1.09 (s, 3 H), 1.22 (s, 3 H), 1.39 (s, 9 H) 1.92 (m, 6 H), 2.30 (m, 5 H), 2.40 (m, 1 H), 2.79 (m, 1 H), 3.44 (s, 1 H), 3.74 (s, 3 H), 3.91 (s, 3 H), 3.95 (d, *J* = 6.4 Hz, 1 H), 4.01 (d, *J* = 1.6 Hz, 1 H), 4.30 (d, *J* = 8.4 Hz, 1 H), 4.42 (d, *J* = 8.4 Hz, 1 H), 4.89 (m, 2 H), 5.24 (m, 2 H), 5.36 (m, 2 H), 5.64 (d, *J* = 6.4 Hz, 1 H), 6.21 (m, 2 H), 6.30 (dd, *J* = 3.2, 1.6 Hz, 1 H), 6.90 (d, *J* = 9.2 Hz, 1 H), 7.02 (dd,*J*= 9.2, 3.2 Hz, 1 H), 7.19 (d, *J* = 3.2 Hz, 1 H), 7.30 (br s, 1 H). m/z LC/MS for C₅₃H₇₄F₃NO₁₉SSiNa⁺: calcd: 1168.4; found:1168.4.

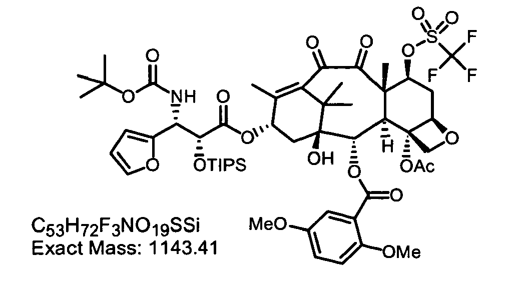

### 7-(trifluoromethanesulfonyloxy)-2'-(triisopropylsilyloxy)-3'-dephenyl-3'-(2-furyl)-10-oxo-2-debenzoyl-2-(2,5-dimethoxybenzoyl)-docetaxel

To a stirred solution of 7-(trifluoromethanesulfonyloxy)-2'-(triisopropylsilyloxy)-3'-dephenyl-3'-(2-furyl)-2-debenzoyl-2-(2,5-dimethoxybenzoyl)-docetaxel (1.44 g, 1.26 mmol) in methylene chloride (30 mL) was added 4-methylmorpholine N-oxide (NMO) (590 mg, 5 mmol) followed by the addition of tetrapropylammonium perruthenate (TPAP) (62 mg, 0.18 mmol). The resulting mixture was allowed to stir at room temperature with monitoring. After 8 hours the reaction was filtered through celite and the pad of celite was rinsed with methylene chloride. The combined supernatent was concentrated in vacuo and the resulting residue was purified on a silica gel column using 10 % ethyl acetate in hexane. The fractions containing the product were pooled and concentrated to give 619 mg of 7-(trifluoromethanesulfonyloxy)-2'-(triisopropylsilyloxy)-3'-dephenyl-3'-(2-furyl)-10-oxo-2-debenzoyl-2-(2,5-dimethoxybenzoyl)-docetaxel as a solid.
¹H NMR (CDCl₃) δ 0.91 (m, 21 H), 1.15 (s, 3 H), 1.25 (s, 3 H), 1.37 (s, 9 H), 1.89 (s, 6 H), 2.14 (m, 1 H), 2.27 (s, 3 H), 2.32 (m, 1 H) 2.51, (m, 1 H), 2.79 (m, 1 H), 3.67 (d,*J*= 6.4 Hz, 1 H), 3.73 (s, 3 H), 3.85 (s, 1 H), 3.91 (s, 3 H), 4.38 (d,*J*= 8.4 Hz, 1 H), 4.40 (d,*J*= 8.4 Hz, 1 H), 4.81 (d,*J*= 8.8 z, 1 H), 4.89 (d,*J*= 1.2 Hz, 1 H), 5.17 (m, 1 H), 5.24 (m, 2 H), 5.72 (d,*J*= 6.0 Hz, 1 H), 6.14 (t, *J* =8.8 Hz, 1 H), 6.20 (d,*J*= 3.2 Hz, 1 H), 6.28 (d,*J*= 3.2, 1.6 Hz, 1 H), 6.90 (d,*J*= 9.2 Hz, 1 H), 7.01 (dd, *J* = 9.2, 3.2 Hz, 1 H), 7.15 (d,*J*= 3.2 Hz, 1 H), 7.29 (br s, 1 H). m/z LC/MS for C₅₃H₇₂F₃NO₁₉SSiNa⁺: calcd: 1166.4; found:1166.1.

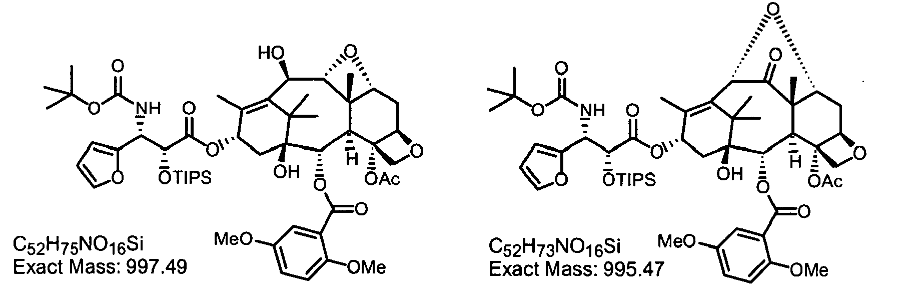

### 7α,9α-epoxy-2'-(triisopropylsilyloxy)-3'-dephenyl-3'-(2-furyl)-2-debenzoyl-2-(2,5-dimethoxybenzoyl)-docetaxel and 7α,10α-epoxy-2'-(triisopropylsilyloxy)-3'-dephenyl-3'-(2-furyl)-2-debenzoyl-2-(2,5-dimethoxybenzoyl)-docetaxel

Sodium borohydride (102 mg, 2.7 mmol) was added to the 7-(trifluoromethanesulfonyloxy)-2'-(triisopropylsilyloxy)-3'-dephenyl-3'-(2-furyl)-10-oxo-2-debenzoyl-2-(2,5-dimethoxybenzoyl)-docetaxel (618 mg, 0.54 mmol) dissolved in ethanol (7.0 mL). After 5 minutes, the reaction was complete and diluted with ethyl acetate. The product was extracted into ethyl acetate (100 mL x 1), washed with saturated aqueous sodium chloride (50 mL x 1), dried over anhydrous sodium sulfate and concentrated *in vacuo.* The crude residue was purified on a silica gel column with 50 % ethyl acetate in hexanes as the eluant, yielding the 7α,9α-epoxy-2'-(triisopropylsilyloxy)-3'-dephenyl-3'-(2-furyl)-2-debenzoyl-2-(2,5-dimethoxybenzoyl)-docetaxel (258 mg, 48 %) and 7α,10α-epoxy-2'-(triisopropylsilyloxy)-3'-dephenyl-3'-(2-furyl)- 2-debenzoyl-2-(2,5-dimethoxybenzoyl)-docetaxel (231 mg, 43 %).

### 7α,9α-epoxy-2'-(triisopropylsilyloxy)-3'-dephenyl-3'-(2-furyl)-2-debenzoyl-2-(2,5-dimethoxybenzoyl)-docetaxel:

¹H NMR (CDCl₃) δ 0.92 (m, 21 H), 1.08 (s, 3 H), 1.23 (s, 3 H), 1.41 (s, 9 H), 1.77 (s, 3 H), 1.99 (s, 3 H), 2.15 (s, 3 H), 2.33 (m, 4 H), 2.56 (d,*J*= 6.8 Hz, 1 H), 3.55 (s, 1 H), 3.76 (s, 3 H), 3.93 (s, 3 H), 4.01 (d,*J*= 6.0 Hz, 1, H), 4.25 (d,*J*= 7.2 Hz, 1 H), 4.32 (d,*J*= 7.2 Hz, 1 H), 4.77 (m, 3 H), 4.92 (br s, 1 H), 4.95 (br s, 1 H), 5.27 (m, 2 H), 5.64 (d,*J*= 6.4 Hz, 1 H), 6.06 (t,*J*= 8.8 Hz, 1 H), 6.20 (d,*J*= 2.8 Hz, 1 H), 6.29 (dd,*J*= 3.2, 2.0 Hz, 1 H), 6.90 (d,*J*= 9.2 Hz, 1 H), 7.00 (dd,*J*= 9.2, 3.2 Hz, 1 H), 7.23 (d,*J*= 3.2 Hz, 1 H), 7.31 (s, 1 H). m/z LC/MS for C₅₂H₇₅NO₁₆SiNa⁺:
calcd: 1020.5; found:1020.4.

### 7α,10α-epoxy-2'-(triisopropylsilyloxy)-3'-dephenyl-3'-(2-furyl)-2-debenzoyl-2-(2,5-dimethoxybenzoyl)-docetaxel:

¹H NMR (CDCl₃) δ 0.95 (m, 21 H), 1.15 (s, 3 H), 1.31 (s, 3 H), 1.39 (s, 9 H), 1.83 (m, 6 H), 2.11 (s, 3 H), 2.20-2.36 (m, 4 H), 3.62 (m, 2 H), 3.75 (s, 3 H), 3.89 (s, 3 H), 4.40 (m, 3 H), 4.78 (br s, 1 H), 4.92 (br s, 1 H), 5.06 (d,*J*= 4.0 Hz, 1 H), 5.25 (m, 2 H), 5.36 (d,*J*= 6.0 Hz, 1 H), 6.18 (m, 2 H), 6.27 (dd,*J*= 2.8, 1.6 Hz, 1 H), 6.88 (d,*J*= 9.2 Hz, 1 H), 6.99 (dd,*J*= 9.2, 3.2 Hz, 1 H), 7.22 (d,*J*= 3.2 Hz, 1 H), 7.29 (br s, 1 H). m/z LC/MS for C₅₂H₇₃NO₁₆SiNa⁺: calcd: 1018.5; found:1018.4.

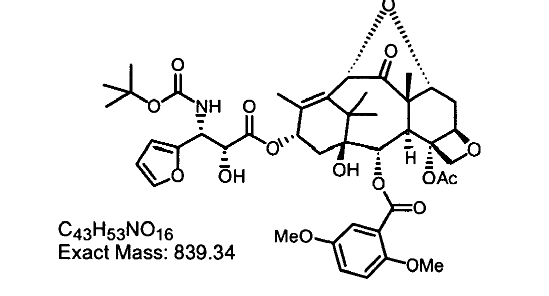

### 7α,10α-epoxy-3'-dephenyl-3'-(2-furyl)-2-debenzoyl-2-(2,5-dimethoxybenzoyl)-docetaxel

7α,10α-epoxy-2'-(triisopropylsilyloxy)-3'-dephenyl-3'-(isobutenyl)-2-debenzoyl-2-(2,5-dimethoxybenzoyl)-docetaxel (49.1 mg, 0.049 mmol) was dissolved in pyridine-acetonitrile (1/1, 2.0 mL) and cooled to 0°C. HF/pyridine (70:30, 0.5 mL) was added and the reaction was allowed to warm to room temperature slowly overnight. The reaction was quenched with saturated aqueous sodium bicarbonate and diluted with ethyl acetate. The ethyl acetate layer was washed with additional saturated aqueous sodium bicarbonate (15 mL x 2) and the combined aqueous layers were then washed with ethyl acetate (40 mL x 2). The combined ethyl acetate layers were washed with water (15 mL x 2), dried over anhydrous sodium sulfate and concentrated *in vacuo.* Crude residue was purified over silica gel with 50 % ethyl acetate in hexanes as the eluant, yielding 7α,10α-epoxy-3'-dephenyl-3'-(2-furyl)-2-debenzoyl-2-(2,5-dimethoxybenzoyl)-docetaxel (33.5 mg, 81 %):
¹H NMR (CDCl₃) δ 1.16 (s, 3 H), 1.30 (s, 3 H), 1.40 (s, 9 H), 1.79 (s, 3 H), 1.87 (s, 3 H), 2.03 (s, 3 H), 2.06 (m, 1 H), 2.34 (m, 3 H), 3.58 (s, 1 H), 3.66 (dd,*J*= 7.2, 2.8 Hz, 1 H), 3.78 (s, 3 H), 3.88 (s, 3 H), 3.93 (d,*J*= 4.0 Hz, 1 H), 4.34 (m, 2 H), 4.49 (d,*J*= 7.6 Hz, 1 H), 4.67 (d,*J*= 2.0 Hz, 1 H), 4.81 (br s, 1 H), 5.04 (d,*J*= 2.0 Hz, 1 H), 5.34 (m, 3 H), 6.18 (br s, 1 H), 6.26 (d,*J*= 3.2 Hz, 1 H), 6.31 (dd,*J*= 3.2, 2.0 Hz, 1 H), 6.92 (d,*J*= 9.2 Hz, 1 H), 7.03 (dd,*J*= 9.2, 3.2 Hz, 1 H), 7.24 (d,*J*= 3.2 z, 1 H), 7.34 (d,*J*= 1.2 Hz, 1 H). m/z LC/MS for C₄₃H₅₃NO₁₆Na⁺: calcd: 862.3; found: 862.3.

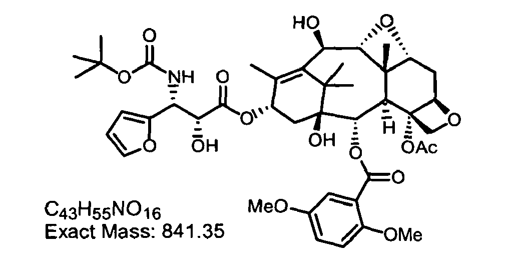

### 7α,9α-epoxy-3'-dephenyl-3'-(2-furyl)-2-debenzoyl-2-(2,5-dimethoxybenzoyl)-docetaxel

7α,9α-epoxy-2'-(triisopropylsilyloxy)-3'-dephenyl-3'-(2-furyl)-2-debenzoyl-2-(2,5-dimethoxybenzoyl)-docetaxel was dissolved in pyridine-acetonitrile (1/1, 1.5 mL) and cooled to 0°C. HF/pyridine (70:30, 0.2 mL) was added and the reaction was allowed to warm to room temperature slowly overnight. The reaction was quenched with saturated aqueous sodium bicarbonate and diluted with ethyl acetate. The ethyl acetate layer was washed with additional saturated aqueous sodium bicarbonate (10 mL x 2). The combined aqueous layers were washed with ethyl acetate (20 mL x 2). The combined ethyl acetate layers were then washed with water (10 mL x 2), dried over anhydrous sodium sulfate and concentrated *in vacuo.* Crude residue was purified over silica gel with 75 % ethyl acetate in hexanes as the eluant yielding 7α,9α-epoxy-3'-dephenyl-3'-(2-furyl)-2-debenzoyl-2-(2,5-dimethoxybenzoyl)-docetaxel as a white solid (10.26 mg, 70 %):
¹H NMR (CDCl₃) δ 1.09 (s, 3 H), 1.23 (s, 3 H), 1.40 (s, 9 H), 1.77 (s, 3 H), 1.95 (s, 3 H), 2.07 (s, 3 H), 2.21 (m, 2 H), 2.37 (m, 2 H), 2.50 (d,*J*= 6.0 Hz, 1 H), 3.51 (s, 1 H), 3.71 (d,*J*= 4.0 Hz, 1 H), 3.77 (s, 3 H), 3.91 (s, 3 H), 4.02 (d,*J*= 6.4 Hz, 1 H), 4.26 (d,*J*= 7.2 Hz, 1 H), 4.29 (d,*J*= 7.2 Hz, 1 H), 4.68 (d,*J*= 2.8 Hz, 1 H), 4.78 (m, 3 H), 4.88 (br s, 1 H), 5.32 (m 2 H), 5.63 (d,*J*= 6.4 Hz, 1 H), 6.01 (t,*J*= 8.8 Hz, 1 H), 6.27 (d,*J*= 3.2 Hz, 1 H), 6.31 (dd,*J*= 3.2, 2.0 Hz, 1 H), 6.92 (d,*J*= 9.2 Hz, 1 H), 7.20 (dd,*J*= 9.2, 3.2 Hz, 1 H), 7.24 (d,*J*= 3.2 Hz, 1 H), 7.34 (d,*J*= 0.8 Hz, 1 H). m/z LC/MS for C₄₃H₅₅NO₁₆Na⁺: calcd: 864.5; found: 864.3.

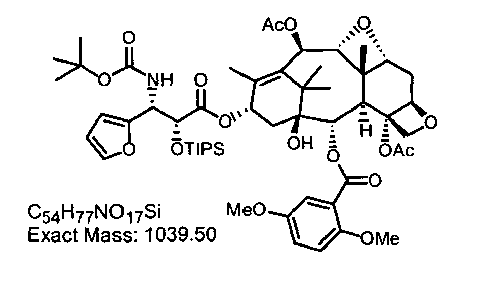

### 7α,9α-epoxy-2'-(triisopropylsilyloxy)-3'-dephenyl-3'-(2-furyl)-2-debenzoyl-2-(2,5-dimethoxybenzoyl)-10-acetoxy-docetaxel

Acetic anhydride (8.1 µL, 0.858 mmol) and DMAP (5 mg, 0.043 mmol) were added to a solution of 7α,9α-epoxy-2'-(triisopropylsilyloxy)-3'-dephenyl-3'-(2-furyl)-2-debenzoyl-2-(2,5-dimethoxybenzoyl)-docetaxel (28.5 mg, 0.0286 mmol) in pyridine (0.5 mL). The reaction was complete after stirring at room temperature for 3 hours. Product was extracted into ethyl acetate (30 mL x 1), washed with water (15 mL x 1) and brine (15 mL x 1), dried over anhydrous sodium sulfate and concentrated *in vacuo.* Crude residue was purified over silica gel with 50 % ethyl acetate in hexanes as the eluant, yielding 7α,9α-epoxy-2'-(triisopropylsilyloxy)-3'-dephenyl-3'-(2-furyl)-2-debenzoyl-2-(2,5-dimethoxybenzoyl)-10-acetoxy-docetaxel as a white solid (25 mg, 85 %)
¹H NMR (CDCl₃) δ 0.95 (m, 21 H), 1.26 (m, 6 H), 1.44 (s, 9 H), 1.76 (s, 3 H), 1.99 (s, 3 H), 2.13 (s, 3 H), 2.18 (s. 3 H), 2.40 (m, 4 H), 3.66 (s, 1 H), 3.80 (s, 3 H), 3.97 (s, 3 H), 4.02 (d,*J*= 6.4 Hz, 1 H), 4.27 (d,*J*= 7.2 Hz, 1 H), 4.35 (d,*J*= 7.2 Hz, 1 H), 4.82 (dd, *J* = 8.4, 6.0 Hz, 1 H), 4.86 (d, *J* = 6.0 Hz, 1 H), 4.95 (br s, 1 H), 5.01 (s, 1 H), 5.31 (s, 2 H), 5.72 (m, 2 H), 6.01 (t, *J* = 8.8 Hz, 1 H), 6.24 (d, *J* = 3.2, 1 H), 6.32 (dd,*J*= 3.2, 1.6 Hz, 1 H), 6.94 (d,*J*= 9.2 Hz, 1 H), 7.05 (dd,*J*= 9.2, 3.2 Hz, 1 H), 7.27 (d,*J*= 3.2, 1 H), 7.34 (d,*J*= 0.8 Hz, 1 H). m/z LC/MS for C₅₄H₇₇NO₁₇SiNa⁺: calcd: 1062.5; found: 1062.5.

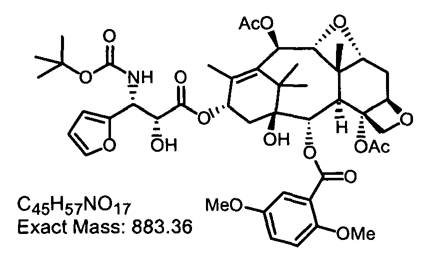

### 7α,9α-epoxy-3'-dephenyl-3'-(2-furyl)-2-debenzoyl-2-(2,5-dimethoxybenzoyl)-10-acetoxy-docetaxel

7α,9α-epoxy-2'-(triisopropylsilyloxy)-3'-dephenyl-3'-(2-furyl)-2-debenzoyl-2-(2,5-dimethoxybenzoyl)-10-acetoxy-docetaxel (25 mg, 0.025 mmol) was dissolved in pyridine-acetonitrile (1/1, 1.5 mL) and cooled to 0°C. HF/pyridine (70:30, 0.25 mL) was added and the reaction was allowed to warm to room temperature slowly overnight. The reaction was quenched with saturated aqueous sodium bicarbonate and diluted with ethyl acetate. The ethyl acetate layer was washed with additional saturated aqueous sodium bicarbonate (10 mL x 2). The combined aqueous layers were washed with ethyl acetate (25 mL x 2). The combined ethyl acetate layers were then washed with water (10 mL x 2), dried over anhydrous sodium sulfate and concentrated *in vacuo.* Crude residue was purified over silica gel with 80 % ethyl acetate in hexanes as the eluant yielding final product, 7α,9α-epoxy-3'-dephenyl-3'-(2-furyl)-2-debenzoyl-2-(2,5-dimethoxybenzoyl)-10-acetoxy-docetaxel (17.2 mg, 78 %):
¹H NMR (CDCl₃) δ 1.24 (s, 6, H), 1.41 (s, 9 H), 1,74 (s, 3 H), 1.94 (s, 3 H), 2.10 (s, 6 H), 2.27 (m, 2 H), 2.33 (m, 1 H), 2.46 (m, 1 H), 3.62 (s, 1 H), 3.79 (s, 4 H), 3.93 (s, 3 H), 4.01 (d,*J*= 6.0 Hz, 1 H), 4.26 (d,*J*= 7.2 Hz, 1 H), 4.30 (d,*J*= 7.2 Hz, 1 H), 4.72 (d,*J*= 3.6 Hz, 1 H), 4.80 (dd,*J*= 5.6, 8.8 Hz, 1 H), 4.85 (d,*J*= 6.0 Hz, 1 H), 4.89 (br s, 1 H), 5.34 (m, 2 H), 5.65 (d,*J*= 6.0 Hz, 1 H), 5.70 (d,*J*= 6.0 Hz, 1 H), 6.02 (t,*J*= 8.8 Hz, 1 H), 6.29 (d,*J*= 3.2 Hz, 1 H), 6.33 (m, 1 H), 6.94 (d,*J*= 9.2 Hz, 1 H), 7.05 (dd,*J*= 9.2, 3.2 Hz, 1 H), 7.26 (d,*J*= 3.2 Hz, 1 H), 7.36 (d,*J*= 1.2 Hz, 1 H). m/z LC/MS for C₄₅H₅₇NO₁₇Na⁺: calcd: 906.4; found: 906.4.

### 3'-isobutenyl series with SS linkers

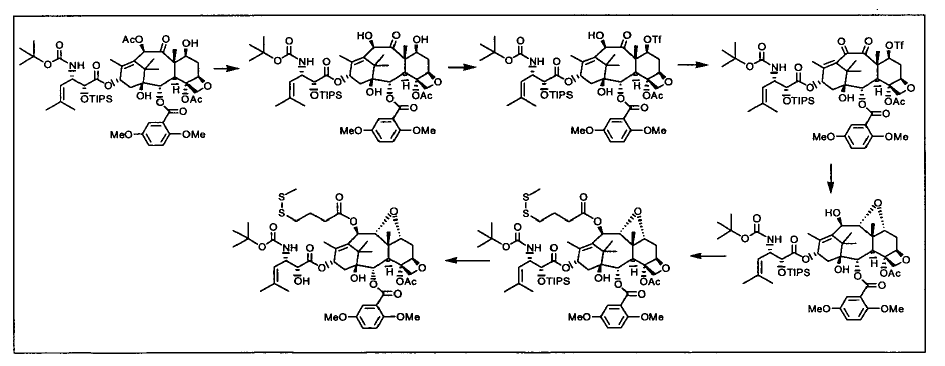

The following intermediates were previously described :

### 2'-(triisopropylsilyloxy)-3'-dephenyl-3'-(isobutenyl)-2-debenzoyl-2-(2,5-dimethoxybenzoyl)-docetaxel

### 7-(trifluoromethanesulfonyloxy)-2'-(triisopropylsilyloxy)-3'-dephenyl-3'-(isobutenyl)-2-debenzoyl-2-(2,5-dimethoxybenzoyl)-docetaxel

### 7-(trifluoromethanesulfonyloxy)-2'-(triisopropylsilyloxy)-3'-dephenyl-3'-(isobutenyl)-10-oxo-2-debenzoyl-2-(2,5-dimethoxybenzoyl)-docetaxel

### 7α,9α-epoxy-2'-(triisopropylsilyloxy)-3'-dephenyl-3'-(isobutenyl)-2-debenzoyl-2-(2,5-dimethoxybenzoyl)-docetaxel.

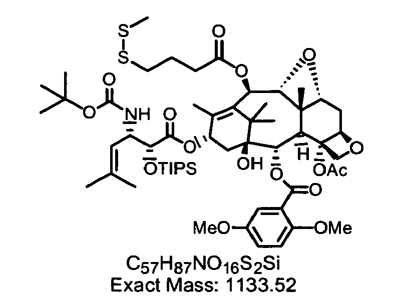

### 7α,9α-epoxy-2'-(triisopropylsilyloxy)-3'-dephenyl-3'-(isobutenyl)-2-debenzoyl-2-(2,5-dimethoxybenzoyl)-10-(4-methyldithiobutanoyl)-docetaxel.

To a solution of 7α,9α-epoxy-2'-(triisopropylsilyloxy)-3'-dephenyl-3'-(isobutenyl)-2-debenzoyl-2-(2,5-dimethoxybenzoyl)-docetaxel (28 mg, 0.029 mmol) dissolved in methylene chloride (1.5 mL) under nitrogen was added DMAP (3.5 mg, 0.028 mmol) and 4-methyldithiobutanoic acid (50 mg, 0.28 mmol). To this mixture was added diisopropylcarbodiimide (0.045 mL, 0.28 mmol) and the resulting mixture was allowed to stir at room temperature overnight. The reaction was then quenched with ammonium chloride (15 mL) and extracted with methylene chloride (20 mL). The organic layer was then washed with brine (15 mL), dried over anhydrous sodium sulfate, filtered, and concentrated in vacuo. The residue was purified by silica ptlc using 40 % ethyl acetate in hexane as the developing solvent. The band containing the desired product was scraped and rinsed with ethyl acetate at the filter. The organic layers were combined and concentrated in vacuo to give 23 mg of 7α,9a-epoxy-2'-(triisopropylsilyloxy)-3'-dephenyl-3'-(isobutenyl)-2-debenzoyl-2-(2,5-dimethoxybenzoyl)-10-(4-methyldithiobutanoyl)-docetaxel as a white solid.
¹H NMR (CDCl₃) δ 1.10 (m, 21 H), 1.21 (s, 3 H), 1.23 (s, 3 H), 1.39 (s, 9 H), 1.68 (s, 3 H), 1.71 (s, 3 H), 1.74 (s, 3 H), 1.95 (s, 3 H), 2.06 (dt,*J*= 2.0, 7.2 Hz, 2 H), 2.08 (s, 3 H), 2.31 (m, 3 H), 2.40 (s, 3 H), 2.49 (m, 3 H), 2.74 (dt, *J* = 2.0, 7.2 Hz, 2 H), 3.80 (s, 3 H), 3.98 (s, 4 H), 4.25 (d, *J* = 7.2 Hz, 1 H), 4.33 (d, *J* = 7.2 Hz, 1 H), 4.46 (d,*J*= 2.0 Hz, 1 H), 4.77 (m, 2 H), 4.84 (d,*J*= 6.4 Hz, 1 H), 4.92 (s, 1 H), 4.92 (d,*J*= 7.2 Hz, 1 H), 5.36 (d,*J*= 8.4 Hz, 1 H), 5.71 (overlapping d,*J*= 6.4 Hz, 2 H), 5.90 (t,*J*= 8.8 Hz, 1 H), 6.95 (d,*J*= 9.2 Hz, 1 H), 7.06 (dd,*J*= 3.2 9.2 Hz, 1 H), 7.30 (d,*J*= 3.2 Hz, 1 H). m/z LC/MS for C₅₇H₈₇NO₁₆S₂SiNa⁺: calcd: 1156.5; found: 1156.2.

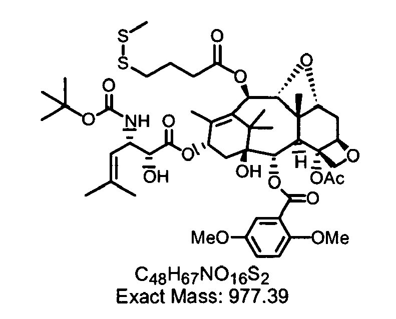

### 7α,9α-epoxy-3'-dephenyl-3'-(isobutenyl)-2-debenzoyl-2-(2,5-dimethoxybenzoyl)-10-(4-methyldithiobutanoyl)-docetaxel

A solution containing 7α,9α-epoxy-2'-(triisopropylsilyloxy)-3'-dephenyl-3'-(isobutenyl)-2-debenzoyl-2-(2,5-dimethoxybenzoyl)-10-(4-methyldithiobutanoyl)-docetaxel (23 mg, 0.02 mmol) dissolved in a 1:1 mixture of acetonitrile:pyridine (1.6 mL) was cooled to 0°C in an ice bath. To this was added hydrogen fluoride-pyridine (0.25 mL) and the solution was allowed to gradually warm to room temperature. After 16 hours the reaction was diluted with ethyl acetate (15 mL) and quenched with a saturated sodium bicarbonate solution (15 mL). The organic layer was washed once with brine (15 mL) and then dried over anhydrous sodium sulfate, filtered, and concentrated in vacuo. The crude residue was purified by silica ptlc using 65 % ethyl acetate in hexane as the developing solvent. The band containing the desired product was scraped and rinsed with ethyl acetate at the filter. The organic layers were combined and concentrated in vacuo to give 16 mg of 7α,9α-epoxy-3'-dephenyl-3'-(isobutenyl)-2-debenzoyl-2-(2,5-dimethoxybenzoyl)-10-(4-methyldithiobutanoyl)-docetaxel.
¹H NMR (CDCl₃) δ 1.25 (s, 3 H), 1.29 (s, 3 H), 1.40 (s, 9 H), 1.73 (s, 6 H), 1.74 (s, 3 H), 1.97 (s, 3 H), 2.04 (m, 2 H), 2.07 (s, 3 H), 2.17 (m, 1 H), 2.25 (m, 1 H), 2.33 (m, 1 H), 2.39 (s, 3 H), 2.51 (m, 1 H), 2.73 (t,*J*= 7.2 Hz, 1 H), 3.79 (s, 3 H), 3.83 (s, 1 H), 3.93 (s, 3 H), 4.00 (d,*J*= 6.0 Hz, 1 H), 4.26 (m, 2 H), 4.31 (d,*J*= 7.6 Hz, 1 H), 4.78 (m, 2 H), 4.84 (d,*J*= 6.0 Hz, 1 H), 4.90 (s, 1 H), 4.96 (d,*J*= 9.2 Hz, 1 H), 5.27 (d,*J*= 8.4 Hz, 1 H), 5.68 (d,*J*= 6.4 Hz, 1 H), 5.70 (d,*J*= 6.0 Hz, 1 H), 6.00 (t,*J*= 8.4 Hz, 1 H), 6.94 (d,*J*= 9.2 Hz, 1 H), 7.05 (dd,*J*= 3.2 9.2 Hz, 1 H), 7.28 (d,*J*= 3.2 Hz, 1 H). m/z LC/MS for C₄₈H₆₇NO₁₆S₂Na⁺: calcd: 1000.4; found: 1000.2.

### 3'-2-furyl series with SS linkers

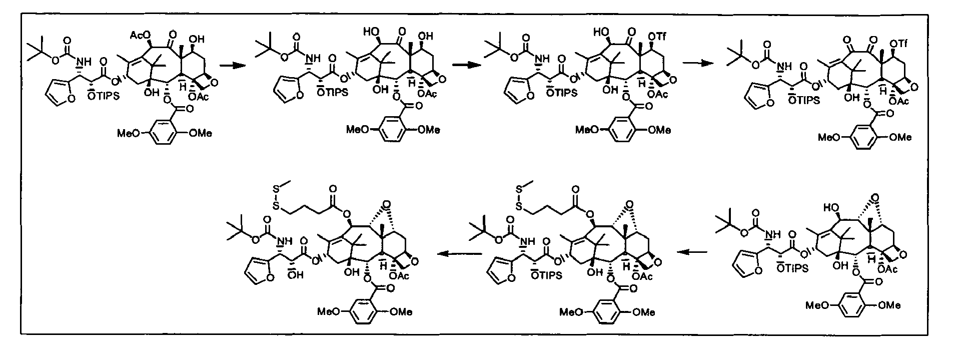

The following intermediates were previously described:

### 2'-(triisopropylsilyloxy)-3'-dephenyl-3'-(2-furyl)-2-debenzoyl-2-(2,5-dimethoxybenzoyl)-docetaxel

### 7-(trifluoromethanesulfonyloxy)-2'-(triisopropylsilyloxy)-3'-dephenyl-3'-(2-furyl)-2-debenzoyl-2-(2,5-dimethoxybenzoyl)-docetaxel

### 7-(trifluoromethanesulfonyloxy)-2'-(triisopropylsilyloxy)-3'-dephenyl-3'-(2-furyl)-10-oxo-2-debenzoyl-2-(2,5-dimethoxybenzoyl)-docetaxel

### 7α,9α-epoxy-2'-(triisopropylsilyloxy)-3'-dephenyl-3'-(2-furyl)-2-debenzoyl-2-(2,5-dimethoxybenzoyl)-docetaxel

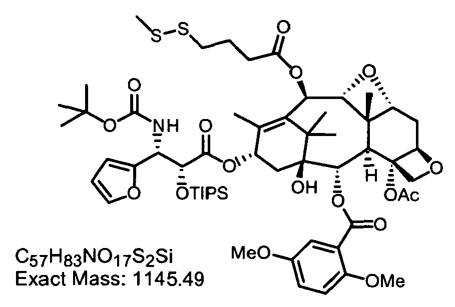

### 7α,9α-epoxy-2'-(triisopropylsilyloxy)-3'-dephenyl-3'-(2-furyl)-2-debenzoyl-2-(2,5-dimethoxybenzoyl)-10-(4-methyldithiobutanoyl)-docetaxel

DMAP (4.6 mg, 0.0374 mmol), 4-methyldithiobutanoic acid (62 mg, 0.374 mmol) and DIC (58.5 µL, .0374 mmol) were added to a solution of 7α,9α-epoxy-2'-(triisopropylsilyloxy)-3'-dephenyl-3'-(2-furyl)-2-debenzoyl-2-(2,5-dimethoxybenzoyl)-docetaxel (37.3 mg, 0.0374 mmol) in methylene chloride (0.5 mL). The reaction was allowed to stir at room temperature overnight, after which it was complete and quenched with saturated aqueous ammonium chloride. Product was extracted into ethyl acetate (25 mL x 2), washed with water (15 mL x 1), dried over magnesium sulfate and concentrated *in vacuo.* The crude residue was purified over silica gel with 50 % ethyl acetate in hexane as the eluant, yielding 7α,9α-epoxy-2'-(triisopropylsilyloxy)-3'-dephenyl-3'-(2-furyl)-2-debenzoyl-2-(2,5-dimethoxybenzoyl)-10-(4-methyldithiobutanoyl)-docetaxel (48.2 mg, 100+ %):
¹H NMR (CDCl₃) δ 0.92 (m, 21 H), 1.24 (m, 6 H), 1.41 (s, 9 H), 1.73 (s, 3 H), 1.95 (s, 3 H), 2.05 (m, 2 H), 2.15 (s, 3 H), 2.32 (m, 3 H), 2.38 (s, 3 H), 2.44-2.56 (m, 3 H), 2.72 (m, 2 H), 3.65 (s, 1 H), 3.77 (s, 3 H), 3.94 (s, 3 H), 3.98 (m, 1 H), 4.26 (m, 2 H), 4.77 (dd,*J*= 8.4, 6.0 Hz, 1 H), 4.83 (d,*J*= 6.4 Hz, 1 H), 4.92 (br s, 1 H), 4.97 (s, 1 H), 5.28 (s, 2 H) 5.68 (m, 2 H), 5.98 (t,*J*= 8.8 Hz, 1 H), 6.20 (d,*J*= 3.2 Hz, 1 H), 6.29 (dd,*J*= 3.2, 2.0 Hz, 1 H), 6.90 (d,*J*= 9.2 Hz, 1 H), 7.01 (dd,*J*= 9.2, 3.2 Hz, 1 H), 7.24 (d,*J*= 3.2 Hz, 1 H), 7.31 (d,*J*= 1.2, 1 H). m/z LC/MS for C₅₇H₈₃NO₁₇S₂SiNa⁺: calcd: 1168.5; found: 1168.4

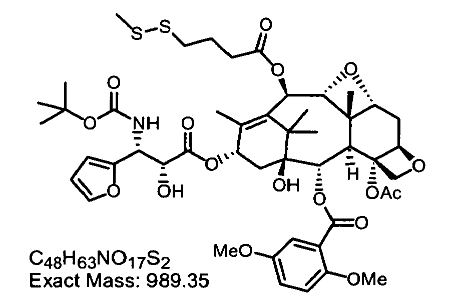

### 7α,9α-epoxy-3'-dephenyl-3'-(2-furyl)-2-debenzoyl-2-(2,5-dimethoxybenzoyl)-10-(4-methyldithiobutanoyl)-docetaxel

7α,9α-epoxy-2'-(triisopropylsilyloxy)-3'-dephenyl-3'-(2-furyl)-2-debenzoyl-2-(2,5-dimethoxybenzoyl)-10-(4-methyldithiobutanoyl)-docetaxel (42.8 mg, 0.0374 mmol) was dissolved in pyridine-acetonitrile (1/1, 3.0 mL) and cooled to 0°C. HF/pyridine (70:30, 0.5 mL) was added and the reaction was allowed to warm to room temperature slowly overnight. The reaction was quenched with saturated aqueous sodium bicarbonate and diluted with ethyl acetate. The ethyl acetate layer was washed with additional saturated aqueous sodium bicarbonate (15 mL x 2) and the combined aqueous layers were then washed with ethyl acetate (40 mL x 2). The combined ethyl acetate layers were washed with water (15 mL x 2), dried over anhydrous sodium sulfate and concentrated *in vacuo.* Crude residue was purified over silica gel with 50 % ethyl acetate in hexanes as the eluant yielding 7α,9α-epoxy-3'-dephenyl-3'-(2-furyl)-2-debenzoyl-2-(2,5-dimethoxybenzoyl)-10-(4-methyldithiobutanoyl)-docetaxel (22.9 mg, 62 %, 2 steps):
¹H NMR (CDCl₃) δ 1.24 (m , 6H), 1.41 (s, 9 H), 1.74 (s, 3 H), 1.93 (s, 3 H), 2.04 (m, 2 H), 2.09 (s, 3 H), 2.24 (m, 2 H), 2.33 (m, 1 H), 2.37 (s, 3 H), 2.42-2.56 (m, 3 H), 2.72 (t,*J*= 6.8 Hz, 2 H), 3.62 (s, 1 H), 3.75 (d,*J*= 8.4 Hz, 1 H), 3.78 (s, 3 H), 3.93 (s, 3 H), 3.99 (d,*J*= 6.0 Hz, 1 H), 4.26 (d,*J*= 7.2 Hz, 1 H), 4.30 (d,*J*= 7.2 Hz, 1 H), 4.70 (d,*J*= 3.6 Hz, 1 H), 4.77 (dd,*J*= 8.8, 5.6 Hz, 1 H), 4.83 (d,*J*= 6.0 Hz, 1 H), 4.88 (br s, 1 H), 5.33 (br s, 2 H), 5.67 (m, 2 H), 6.01 (t,*J*= 8.8 Hz, 1 H), 6.28 (d, J = 3.2, 1 H), 6.32 (dd,*J*= 3.2, 2.0 Hz, 1 H), 6.92 (d,*J*= 9.2 Hz, 1 H), 7.03 (dd,*J*= 9.2, 3.2 Hz, 1 H), 7.25 (d,*J*= 3.2, 1 H), 7.35 (m, 1 H). m/z LC/MS for C₄₈H₆₃NO₁₇S₂Na⁺: calcd: 1012.3; found: 1012.3.

The activity of the compounds of the present invention were determined following the proceeding described by Riou, Naudin and Lavelle in Biochemical and Biophysical Research Communications; Vol. 187, No1, 1992, p 164-170.

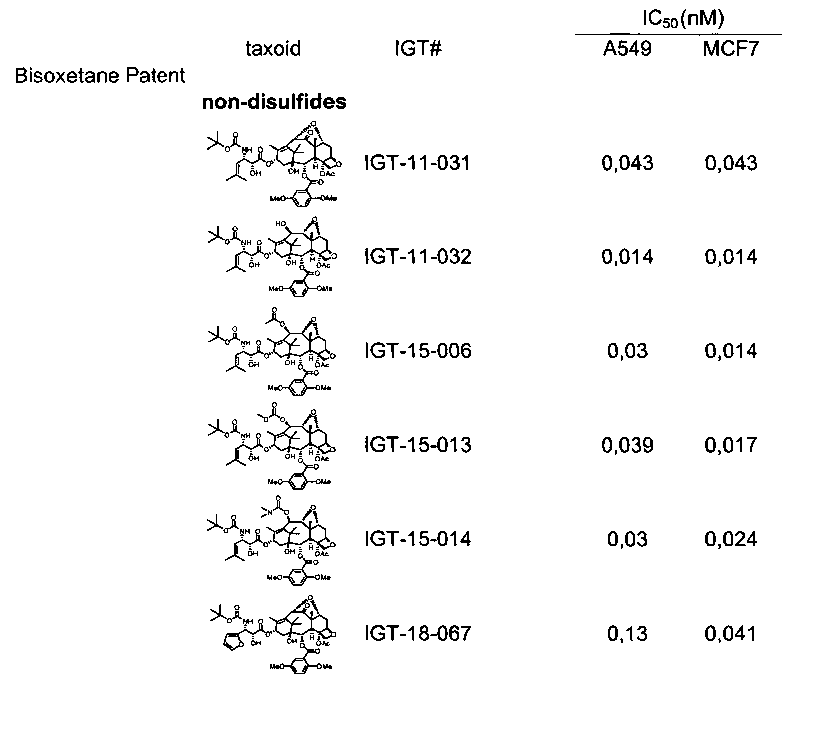

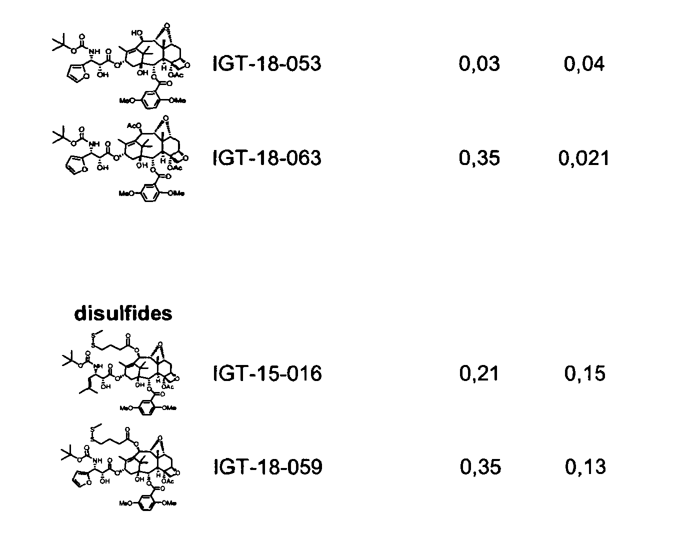

## Claims

1. Taxanes having the following formula (I) : 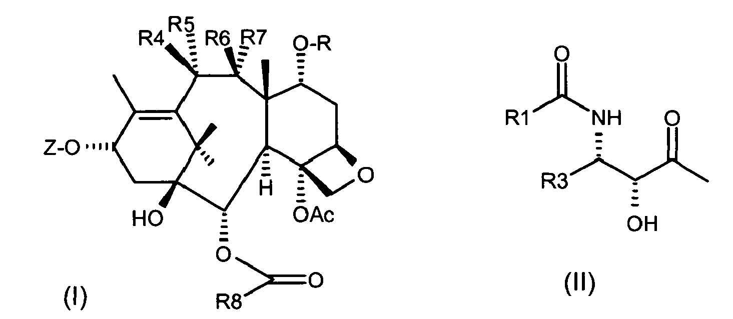 wherein **Z** = H or a radical of formula **II**
**R**_{**1**} is a linker or an optionally substituted aryl or heterocyclic radical, alkyl having from 1 to 10 carbon atoms, alkenyl or alkynyl having from 2 to 10 carbon atoms, cycloalkyl or cycloalkenyl having from 3 to 10 carbon atoms, -OR₂ or a carbamate formed from any of said alkyl having from 1 to 10 carbon atoms, alkenyl or alkynyl having from 2 to 10 carbon atoms, cycloalkyl or cycloalkenyl having from 3 to 10 carbon atoms, or optionally substituted aryl or heterocyclyl radical.
**R**_{**2**} is an alkyl radical having from 1 to 10 carbon atoms, alkenyl or alkynyl having from 2 to 10 carbon atoms, cycloalkyl or cycloalkenyl having from 3 to 10 carbon atoms, optionally substituted aryl or heterocyclic radical.
**R**_{**3**} is a linker or an optionally substituted aryl or heterocyclic radical, alkyl having from 1 to 10 carbon atoms, alkenyl or alkynyl having from 2 to 10 carbon atoms, cycloalkyl or cycloalkenyl having from 3 to 10 carbon atoms
**R**_{**4**} is a linker, H, a hydroxy radical, an alkoxy, an alkenyloxy, an optionally substituted alkanoyloxy, aroyloxy, alkenoyloxy, alkynoyloxy, cycloalkanoyloxy, alkoxyacetyl, alkylthioacetyl, alkyloxycarbonyloxy, cycloalkyloxy, cycloalkenyloxy, carbamoyloxy, alkylcarbamoyloxy, or dialkylcarbamoyloxy, a heterocyclic or aryl ether, ester or carbamate, or, a linear, branched, or cyclic alkyl or alkenyl ester or ether having from1 to 10 carbon atoms or a carbamate of the formula -OCOX,
wherein X is a nitrogen-containing heterocycle such as unsubstituted or substituted piperidino, morpholino, piperazino, N-methylpiperazino or a carbamate of the formula -OCON**R**_{**9**}**R**_{**10**}, wherein **R**_{**9**} and **R**_{**10**} are the same or different and are H, linear, branched, or cyclic alkyl having from 1 to 10 atoms or simple or substituted aryl having from 1 to 10 carbon atoms.
**R**_{**5**} or **R**_{**7**} is H
**R**_{**6**} is a H
**R**_{**7**} or **R**_{**5**} and **R** form a bond (cyclic ether)
**R**_{**8**} = optionally substituted aryl or heterocyclic radical

2. Compounds of formula (I) wherein R₁ is -OR₂ or an optionally substituted aryl or a heterocyclic radical.

3. Compounds of formula (I) wherein R₂ is an alkyl group and more preferably a substituted alkyl group such as a terbutyl group.

4. Compounds of formula (I) wherein R₄ is a linker or an alkanoyloxy radical.

5. Compounds of claim 1 wherein **R**_{**1**} is chosen among the following substituents t-butoxy, crotyl, dimethylacrylyl, isobutenyl, hexenyl, cyclopentenyl, cyclohexenyl, furyl, pyrollyl, thienyl, thiazolyl, imidazolyl, pyridyl, oxazolyl, indolyl, benzofuranyl or benzothienyl.

6. Compounds of claim 5 wherein **R**_{**1**} is t-butoxy, isobutenyl, crotyl, dimethyacrylyl, thienyl, thiazolyl or furyl

7. Compounds of claim 1 wherein **R**_{**3**} is chosen among the following substituents crotyl, dimethylacrylyl, propenyl, isobutenyl, hexenyl, cyclopentenyl, cyclohexenyl, furyl, pyrollyl, thienyl, thiazolyl, imidazolyl, pyridyl, morpholino, piperidino, piperazino, oxazolyl, indolyl, benzofuranyl or benzothienyl.

8. Compounds of claim 7 wherein **R**_{**3**} is iso-butenyl, crotyl, dimethyacrylyl, thienyl, thiazolyl, pyridyl, tert-butyl, or furyl.

9. Compounds of claim 1 wherein the linking groups are containing disulfide groups, thioether groups, acid labile groups, photolabile groups, peptidase labile groups and esterase labile groups.

10. Compounds of claim 9 wherein the linking group contains disulfide groups and thioether groups.

11. Compounds of claim 10 wherein the linking group is a thiol- or disulfide-containing group, the side chain carrying the thiol or disulfide group can be linear or branched, aromatic or heterocyclic.

12. Compounds of claim 11 wherein the linking group at R1 is choosen among the following substituents -(CR₁₃R₁₄)ₘ(CR₁₅R₁₆)ₙOCH₂CH₂)_{y}SZ', -O(CR₁₃R₁₄)ₘ(CR₁₅R₁₆)ₙ (OCH₂CH₂)_{y} SZ', -(CR₁₃R₁₄)ₘ (CR₁₇=CR₁₈)(CR₁₅R₁₆)ₘ (OCH₂CH₂)_{y}SZ', -O-(CR₁₃R₁₄)ₘ (CR₁₇=CR₁₈)(CR₁₅R₁₆)ₘ (OCH₂CH₂)_{y}SZ', -NR₁₂(CR₁₃R₁₄)ₘ(CR₁₅R₁₆)ₙ (OCH₂CH₂)_{y}SZ', phenyl-X'SZ', furyl-XSZ', oxazolyl-X'SZ', thiazolyl-X'SZ', thienyl-X'SZ', imidazolyl-X'SZ', morpholino-X'SZ', -piperazino-X'SZ', piperidino-XSZ', -furyl-X'SZ', -thienyl-X'SZ', -thiazolyl-X'SZ' and --N-methylpiperazino-X'SZ', -morpholino-X'SZ', -piperazino-X'SZ', -piperidino-X'SZ', or -N-methylpiperazino-X'SZ', wherein:
Z' is H or SR',
and wherein -X' is a direct link or a linear alkyl or branched alkyl having from 1-10 carbon atoms or a polyethylene glycol spacer with 2 to 20 repeating ethylene oxy units;
R' and R₁₂ are the same or different and are linear alkyl, branched alkyl or cyclic alkyl having from 1 to 10 carbon atoms, or simple or substituted aryl or heterocyclic, and R₁₂ can in addition be H,
R₁₃, R₁₄, R₁₅ and R₁₆ are the same or different and are H or a linear or branched alkyl having from 1 to 4 carbon atoms,
R₁₇ and R₁₈ are H or alkyl,
n is an integer of 1 to 10,
m is an integer from 1 to 10 and can also be 0,
y is an integer from 1 to 20 and can also be 0.

13. Compounds of claim 11 wherein the linking group at R4 is choosen among the following substituents -O(CR₁₃R₁₄)ₘ(CR₁₅R₁₆)ₙOCH₂CH₂)_{y}SZ', -OCO(CR₁₃R₁₄)ₘ(CR₁₅R₁₆)ₙ (OCH₂CH₂)_{y} SZ', -O(CR₁₃R₁₄)ₘ (CR₁₇=CR₁₈)(CR₁₅R₁₆), (OCH₂CH₂)_{y}SZ', -OCO-(CR₁₃R₁₄)ₘ (CR₁₇=CR₁₈)(CR₁₅R₁₆)ₘ (OCH₂CH₂)_{y}SZ', -OCONR₁₂(CR₁₃R₁₄)ₘ(CR₁₅R₁₆)ₙ (OCH₂CH₂)_{y}SZ', -OCO-phenyl-X'SZ', -OCO-furyl-X'SZ', -OCO-oxazolyl-X'SZ', -OCO-thiazolyl-X'SZ', -OCO-thienyl-X'SZ', -OCO-imidazolyl-X'SZ', -OCO-morpholino-X'SZ', -OCO-piperazino-X'SZ', -OCO-piperidino-X'SZ', and -OCO-N-methylpiperazino-X'SZ', or -OCO-N-methylpiperazino-X'SZ', wherein:
Z' is H or SR',
wherein X' is a direct link or a linear alkyl or branched alkyl having from 1-10 carbon atoms or a polyethylene glycol spacer with 2 to 20 repeating ethylene oxy units;
R' and R₁₂ are the same or different and are linear alkyl, branched alkyl or cyclic alkyl having from 1 to 10 carbon atoms, or simple or substituted aryl or heterocyclic, and R₁₂ can in addition be H,
R₁₃, R₁₄, R₁₅ and R₁₆ are the same or different and are H or a linear or branched alkyl having from 1 to 4 carbon atoms,
R₁₇ and R₁₈ are H or alkyll,
n is an integer of 1 to 10,
m is an integer from 1 to 10 and can also be 0,
y is an integer from 1 to 20 and can also be 0.

14. Compounds of claim 11 wherein the linking group at R3 is choosen among the following substituents -(CR₁₃R₁₄)ₘ(CR₁₅R₁₆)ₙ(OCH₂CH₂)_{y}SZ', -(CR₁₃R₁₄)ₘ (CR₁₇=CR₁₈)(CR₁₅R₁₆)ₘ (OCH₂CH₂)_{y}SZ', phenyl-X'SZ', furyl-X'SZ', oxazolyl-X'SZ', thiazolyl-X'SZ', thienyl-X'SZ', imidazolyl-X'SZ', wherein :
Z' is H or SR',
wherein X' is a direct link or a linear alkyl or branched alkyl having from 1-10 carbon atoms or a polyethylene glycol spacer with 2 to 20 repeating ethylene oxy units ;
R' is linear alkyl, branched alkyl or cyclic alkyl having from 1 to 10 carbon atoms, or simple or substituted aryl or heterocyclic,
R₁₃, R₁₄, R₁₅ and R₁₆ are the same or different and are H or a linear or branched alkyl having from 1 to 4 carbon atoms,
R₁₇ and R₁₈ are H or alkyl,
n is an integer of 1 to 10,
m is an integer from 1 to 10 and can also be 0,
y is an integer from 1 to 20 and can also be 0.

15. Compounds of claim 1 wherein **R**_{**8**} is 3-methoxyphenyl, 3-chlorophenyl, 2,5-dimethoxyphenyl, furyl, pyrollyl, thienyl, thiazolyl, imidazolyl, pyridyl, oxazolyl, indolyl, benzofuranyl or benzothienyl.

16. Compounds of claim 1 wherein R₅ is H and R and R₇ form a bound.

17. Compounds of claim 1 wherein in the compound of formula (I)
**R**_{**4**} is the linker
**Z** = H or radical of formula II
**R**_{**1**} is an optionally substituted aryl or heterocyclic radical, alkyl having from 1 to 10 carbon atoms, alkenyl or alkynyl having from 2 to 10 carbon atoms, cycloalkyl or cycloalkenyl having from 3 to 10 carbon atoms, -OR₂ or a carbamate formed from any of said alkyl having from 1 to 10 carbon atoms, alkenyl or alkynyl having from 2 to 10 carbon atoms, cycloalkyl or cycloalkenyl having from 3 to 10 carbon atoms, or optionally substituted aryl or heterocyclyl radical
**R**_{**2**} is an alkyl radical having from 1 to 10 carbon atoms, alkenyl or alkynyl having from 2 to 10 carbon atoms, cycloalkyl or cycloalkenyl having from 3 to 10 carbon atoms, optionally substituted aryl or heterocyclic radical
**R**_{**3**} is optionally substituted aryl or heterocyclic radical, alkyl having from 1 to 10 carbon atoms, alkenyl or alkynyl having from 2 to 10 carbon atoms, cycloalkyl or cycloalkenyl having from 3 to 10 carbon atoms
**R**_{**4**} is the linking group
**R**_{**5**} or **R**_{**7**} is H
**R**_{**6**} is H
R_{**7**} or **R**_{**5**} and **R** form a bond (cyclic ether)
**R**_{**8**} is an optionally substituted aryl or heterocyclic radical

18. Compounds of claim 1 wherein
**Z** is a radical of formula II
**R**_{**1**} is the linking group
**R**_{**3**} is optionally substituted aryl or heterocyclic radical, alkyl having from 1 to 10 carbon atoms, alkenyl or alkynyl having from 2 to 10 carbon atoms, cycloalkyl or cycloalkenyl having from 3 to 10 carbon atoms
**R**_{**4**} is H, a hydroxy radical, an alkoxy, an alkenyloxy, an optionally substituted alkanoyloxy, aroyloxy, alkenoyloxy, alkynoyloxy, cycloalkanoyloxy, alkoxyacetyl, alkylthioacetyl, alkyloxycarbonyloxy, cycloalkyloxy, cycloalkenyloxy, carbamoyloxy, alkylcarbamoyloxy, or dialkylcarbamoyloxy, a heterocyclic or aryl ether, ester or carbamate, or, a linear, branched, or cyclic alkyl or alkenyl ester or ether having from 1 to 10 carbon atoms or a carbamate of the formula -OCOX, wherein X is a nitrogen-containing heterocycle such as unsubstituted or substituted piperidino, morpholino, piperazino, N-methylpiperazino, or a carbamate of the formula -OCON**R**_{**9**}**R**_{**10**}**,** wherein **R**_{**9**} and **R**_{**10**} are the same or different and are H, linear, branched, or cyclic alkyl having from 1 to 10 atoms or simple or substituted aryl having from 5 to 10 carbon atoms;
**R**_{**5**} or **R**_{**7**} is H
**R**_{**6**} is H
**R**_{**7**} or **R**_{**5**} and **R** form a bond (cyclic ether)
**R**_{**8**} is an optionally substituted aryl or heterocyclic radical

19. Compounds of claim 1
wherein **R**_{**3**} is the linker
Z is a radical of formula II
**R**_{**1**} is an optionally substituted aryl or heterocyclic radical, alkyl having from 1 to 10 carbon atoms, alkenyl or alkynyl having from 2 to 10 carbon atoms, cycloalkyl or cycloalkenyl having from 3 to 10 carbon atoms, **-OR**_{**2**} or a carbamate formed from any of said alkyl having from 1 to 10 carbon atoms, alkenyl or alkynyl having from 2 to 10 carbon atoms, cycloalkyl or cycloalkenyl having from 3 to 10 carbon atoms, or optionally substituted aryl or heterocyclyl radical
**R**_{**2**} is alkyl radical having from 1 to 10 carbon atoms, alkenyl or alkynyl having from 2 to 10 carbon atoms, cycloalkyl or cycloalkenyl having from 3 to 10 carbon atoms, optionally substituted aryl or heterocyclic radical
**R**_{**3**} is the linking group
**R**_{**4**} is H, a hydroxy radical, an alkoxy, an alkenyloxy, an optionally substituted alkanoyloxy, aroyloxy, alkenoyloxy, alkynoyloxy, cycloalkanoyloxy, alkoxyacetyl, alkylthioacetyl, alkyloxycarbonyloxy, cycloalkyloxy, cycloalkenyloxy, carbamoyloxy, alkylcarbamoyloxy, or dialkylcarbamoyloxy, a heterocyclic or aryl ether, ester or carbamate, or, a linear, branched, or cyclic alkyl or alkenyl ester or ether having from 1 to 10 carbon atoms or a carbamate of the formula -OCOX, wherein X is a nitrogen-containing heterocycle such as unsubstituted or substituted piperidino, morpholino, piperazino, N-methylpiperazino, or a carbamate of the formula -OCON**R**_{**9**}**R**_{**10,**} wherein **R**_{**9**} and **R**_{**10**} are the same or different and are H, linear, branched, or cyclic alkyl having from 1 to 10 atoms or simple or substituted aryl having from 5 to 10 carbon atoms;
**R**_{**5**} or **R**_{**7**} is H
**R**_{**6**} is H
**R**_{**7**} or **R**_{**5**} and **R** form a bond (cyclic ether)
**R**_{**8**} is an optionally substituted aryl or heterocyclic radical

20. A cytotoxic agent comprising one or more taxanes covalently bonded to a cell binding agent through a linking group, wherein at least one of said taxanes is a compound represented by formula (III): 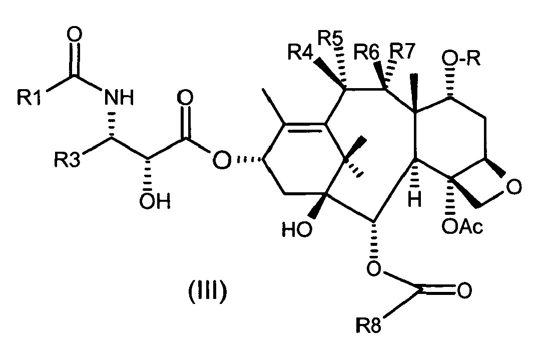 wherein:
**R**_{**1**} is a linker
**R**_{**2**} is an alkyl radical having from 1 to 10 carbon atoms, alkenyl or alkynyl having from 2 to 10 carbon atoms, cycloalkyl or cycloalkenyl having from 3 to 10 carbon atoms, optionally substituted aryl or heterocyclic radical
**R**_{**3**} is an optionally substituted aryl or heterocyclic radical, alkyl having from 1 to 10 carbon atoms, alkenyl or alkynyl having from 2 to 10 carbon atoms, cycloalkyl or cycloalkenyl having from 3 to 10 carbon atoms
**R**_{**4**} is H, a hydroxy radical, an alkoxy, an alkenyloxy, an optionally substituted alkanoyloxy, aroyloxy, alkenoyloxy, alkynoyloxy, cycloalkanoyloxy, alkoxyacetyl, alkylthioacetyl, alkyloxycarbonyloxy, cycloalkyloxy, cycloalkenyloxy, carbamoyloxy, alkylcarbamoyloxy, or dialkylcarbamoyloxy, a heterocyclic or aryl ether, ester or carbamate, or, a linear, branched, or cyclic alkyl or alkenyl ester or ether having from 1 to 10 carbon atoms or a carbamate of the formula -OCOX, wherein X is a nitrogen-containing heterocycle such as unsubstituted or substituted piperidino, morpholino, piperazino, N-methylpiperazino, or a carbamate of the formula -OCON**R**_{**9**}**R**_{**10**}, wherein **R**_{**9**} and **R**_{**10**} are the same or different and are H, linear, branched, or cyclic alkyl having from 1 to 10 atoms or simple or substituted aryl having from 1 to 10 carbon atoms.
**R**_{**5**} or **R**_{**7**} is H
**R**_{**6**} is H
**R**_{**7**} or **R**_{**5**} and **R** form a bond (cyclic ether)
**R**_{**8**} = optionally substituted aryl or heterocyclic radical

21. A cytotoxic agent comprising one or more taxanes covalently bonded to a cell binding agent through a linking group, wherein at least one of said taxanes is a compound represented by formula (III): 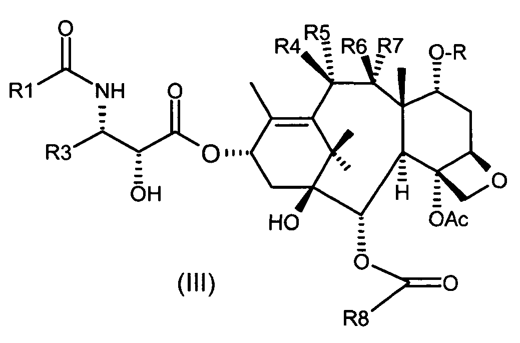 wherein:
**R**_{**1**} is an optionally substituted aryl or heterocyclic radical, alkyl having from 1 to 10 carbon atoms, alkenyl or alkynyl having from 2 to 10 carbon atoms, cycloalkyl or cycloalkenyl having from 3 to 10 carbon atoms, -OR₂ or a carbamate formed from any of said alkyl having from 1 to 10 carbon atoms, alkenyl or alkynyl having from 2 to 10 carbon atoms, cycloalkyl or cycloalkenyl having from 3 to 10 carbon atoms, or optionally substituted aryl or heterocyclyl radical
**R**_{**2**} is an alkyl radical having from 1 to 10 carbon atoms, alkenyl or alkynyl having from 2 to 10 carbon atoms, cycloalkyl or cycloalkenyl having from 3 to 10 carbon atoms, optionally substituted aryl or heterocyclic radical
**R**_{**3**} is a linker
**R**_{**4**} is H, a hydroxy radical, an alkoxy, an alkenyloxy, an optionally substituted alkanoyloxy, aroyloxy, alkenoyloxy, alkynoyloxy, cycloalkanoyloxy, alkoxyacetyl, alkylthioacetyl, alkyloxycarbonyloxy, cycloalkyloxy, cycloalkenyloxy, carbamoyloxy, alkylcarbamoyloxy, or dialkylcarbamoyloxy, a heterocyclic or aryl ether, ester or carbamate, or, a linear, branched, or cyclic alkyl or alkenyl ester or ether having from 1 to 10 carbon atoms or a carbamate of the formula -OCOX, wherein X is a nitrogen-containing heterocycle such as unsubstituted or substituted piperidino, morpholino, piperazino, N-methylpiperazino, or a carbamate of the formula -OCON**R**_{**9**}**R**_{**10**}, wherein **R**_{**9**} and **R**_{**10**} are the same or different and are H, linear, branched, or cyclic alkyl having from 1 to 10 atoms or simple or substituted aryl having from 1 to 10 carbon atoms.
**R**_{**5**} or **R**_{**7**} is H
**R**_{**6**} is H
**R**_{**7**} or **R**_{**5**} and **R** form a bond (cyclic ether)
**R**_{**8**} = optionally substituted aryl or heterocyclic radical

22. A cytotoxic agent comprising one or more taxanes covalently bonded to a cell binding agent through a linking group, wherein at least one of said taxanes is a compound represented by formula (III): 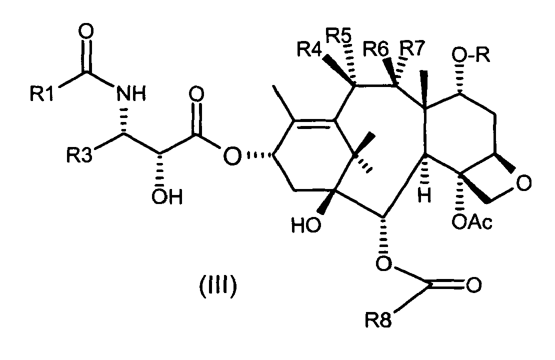 wherein:
**R**_{**1**} is an optionally substituted aryl or heterocyclic radical, alkyl having from 1 to 10 carbon atoms, alkenyl or alkynyl having from 2 to 10 carbon atoms, cycloalkyl or cycloalkenyl having from 3 to 10 carbon atoms, -OR₂ or a carbamate formed from any of said alkyl having from 1 to 10 carbon atoms, alkenyl or alkynyl having from 2 to 10 carbon atoms, cycloalkyl or cycloalkenyl having from 3 to 10 carbon atoms, or optionally substituted aryl or heterocyclyl radical.
**R**_{**2**} is an alkyl radical having from 1 to 10 carbon atoms, alkenyl or alkynyl having from 2 to 10 carbon atoms, cycloalkyl or cycloalkenyl having from 3 to 10 carbon atoms, optionally substituted aryl or heterocyclic radical.
**R**_{**3**} is an optionally substituted aryl or heterocyclic radical, alkyl having from 1 to 10 carbon atoms, alkenyl or alkynyl having from 2 to 10 carbon atoms, cycloalkyl or cycloalkenyl having from 3 to 10 carbon atoms
**R**_{**4**} is a linker
**R**_{**5**} or **R**_{**7**} is H
**R**_{**6**} is H
**R**_{**7**} or **R**_{**5**} and **R** form a bond (cyclic ether)
**R**_{**8**} = optionally substituted aryl or heterocyclic radical

23. A therapeutic composition comprising:
(C) a therapeutically effective amount of the cytotoxic agent of any one of claims 19, 20, or 21; and
(D) a pharmaceutically acceptable carrier.

24. The cytotoxic agent of any one of claims 19, 20, or 21 wherein the cell binding agent is selected from the group consisting of antibodies, an antibody fragment, interferons, lymphokines, hormones, vitamins, growth factors, colony stimulating factors, and transferrin.

25. The cytotoxic agent of any one of claims 19, 20, or 21 wherein the cell binding agent is an antibody.

26. The cytotoxic agent of any one of claims 19, 20, or 21 wherein the cell binding agent is a monoclonal antibody.

27. The cytotoxic agent of any one of claims 19, 20, or 21 wherein the cell binding agent is an antigen specific antibody fragment.

28. The cytotoxic agent of any one of claims 19, 20, or 21 wherein the antibody fragment is sFV, Fab, Fab', or F(ab')₂.

29. The cytotoxic agent of any one of claims 19, 20, or 21 wherein the cell binding agent is a growth factor or colony stimulating factor.

30. A method of killing selected cell populations comprising contacting target cells or tissue containing target cells with an effective amount of the cytotoxic agent of any one of claims 20, 21, or 22.
